# EUROPEAN PATENT SPECIFICATION

(11) **EP 2 500 435 B1**
(45) Date of publication and mention of the grant of the patent: **21.09.2016**
(21) Application number: 11158836.4
(22) Date of filing: 18.03.2011
(51) Int. Cl.: C12Q 1/18, G01N 33/569, C12Q 1/68, C12N 15/82

(54) **Identification of antibiotic resistance in micro organisms**
Nachweis von Antibiotikaresistenz in Mikroorganismen
Identification de résistance antibiotique dans les micro-organismes

(43) Date of publication of application: 19.09.2012
(73) Proprietor: miacom Diagnostics GmbH, 40225 Düsseldorf (DE)
(72) Inventor: Freiherr von Stein, Walter, 40227 Düsseldorf (DE); Stange, Mirko, 40213 Düsseldorf (DE)
(74) Representative: Remus, Alvaro Johannes

(56) References cited:
- WO-A1-2009/095258
- WO-A1-2010/149159

## Description

Subject of the present invention is a method for the simultaneous identification of an individual bacterial cell and detection of an antibiotic resistance in said individual bacterial cell in a biological sample, comprising contacting said individual bacterial cell with a first nucleic acid capable of selectively hybridizing with a nucleic acid in said bacterial cell under *in situ* conditions, wherein the first nucleic acid selectively hybridizes with the nucleic acid, and wherein the first nucleic acid is labelled with a first label, and identifying the bacterial cell by the detection of the presence of the first label in said individual bacterial cell.

The characterisation of micro-organisms in routine diagnostic procedures encompasses the determination of a species' identity and its sensitivity towards antibiotics. In order to achieve this, micro-organisms need to be taken from their environment and enriched in a selective environment for the separate identification (ID) and antibiotic sensitivity testing (AST). Currently the AST/ID of micro-organisms is achieved by identifying presence or absence of an array of biochemical features and the (non-) capability to grow in the presence of antibiotics. Alternatively DNA can, be extracted from a sample and the then pooled DNA is tested for the presence/absence of specific sequences utilising gene amplification techniques. This can signal the presence of an organism in the sample. Equally, the presence of a gene coding for antibiotic resistance in the sample can be detected. By definition, extracting DNA directly from a sample renders pooled DNA from an unknown mixture of cells. Unequivocal results can only be achieved if the DNA is extracted from a pure colony.

Resistance of micro-organisms against antibiotics can be mediated by one of the following mechanisms:
1. Some antibiotics disturb cell wall synthesis in a micro-organism. Resistance against such antibiotics can be mediated by altering the target of the antibiotic, namely a cell wall protein. For example, MRSA or/and ORSA produce an atypical Penicillin Binding Protein (PBP2a), which has a reduced penicillin binding capacity, or does not bind penicillin.
2. The antibiotic is inactivated before reaching the target. An example of this resistance mechanism is the presence of enzymes capable of inactivating the antibiotic by cleavage. For example, beta-lactamase is capable of cleaving the beta-lactam of penicillin or/and carbapenems. Another example is inactivation by binding to a protein so that.the antibiotic can no more reach its target.
3. The antibiotic is removed from the cell before reaching the target by a specific pump. An example is the RND transporter.
4. Some antibiotics act by binding to ribosomal. RNA (rRNA) and interact with protein biosynthesis in the micro-organism. A micro-organism resistant against such antibiotic may comprise a mutated rRNA having a reduced binding capability to the antibiotic, but,having an essentially normal function within the ribosome.

*Staphylococcus aureus* is one of the most common causes of nosocomial or community-based infections, leading to serious illnesses with high rates of morbidity and mortality. In recent years, the increase in the number of bacterial strains that show resistance to methicillin or/and Oxacillin, methicillin resistant *Staphylococcus aureus* (MRSA) and oxacillin resistant *Staphylococcus aureus* (ORSA) have become a serious clinical and epidemiological problem because these antibiotics (or analogues) are considered the first option in the treatment of staphylococci infections. The resistance to these antibiotics implies resistance to many ß-lactam antibiotics, in particular with low affinity for penicillins. For these reasons, accuracy and promptness in the detection of methicillin resistance or/and oxacillin resistance is of key importance to ensure correct antibiotic treatment in infected patients as well as control of MRSA or/and ORSA isolates is hospital environments, to avoid them spreading.

Methicillin-resistant *Staphylococcus aureus* are also termed multiple-resistant *Staphylococcus aureus* (MRSA), multidrug-resistant *Staphylococcus aureus* (MRSA). Methicillin resistance largely overlaps with oxacillin resistance. In other words, a MRSA may be an oxacillin-resistant *Staphylococcus aureus* (ORSA), and vice versa.

MRSA or/and ORSA strains harbour the *mecA* gene, which encoders a modified PBP2 protein (termed PBP2' or PBP2a) with low affinity for methicillin and many ß-lactam antibiotics, in particular with low affinity for penicillins. Phenotypic expression of methicillin resistance may depend on the growth conditions for S. *aureus,* such as temperature or osmolarity of the medium, and this may affect the accuracy of the methods used to detect methicillin resistance (1). Hetero-resistant bacterial strains may evolve into fully resistant strains and therefore be selected in those patients receiving ß-lactam antibiotics, thus causing therapeutic failure. From a clinical point of view they should, therefore, be considered fully resistant.

There are several methods for detecting methicillin resistance (1,9) including classical methods for determining a minimum inhibitory concentration MIC (disc diffusion, Etest, or broth dilution), screening techniques with solid culture medium containing oxacillin, and methods that detect the *mecA* gene or its protein product (PBP2' protein) (3,4). Detection of the *mecA* gene is considered as the reference method for determining resistance to methicillin (1). However, many laboratories throughout the world do not have the funds required, the capacity or the experienced staff required to provide molecular assays for detecting MRSA or/and ORSA isolates. It is therefore essential that other, more useful, screening methods are incorporated into routine clinical practice. Moreover, the presence of antibiotic resistance has it's relevance at several levels, all of which are of clinical significance
1. Presence of a gene conveying resistance, such as *mecA_{;} mef(E)*
2. Presence of a repressor gene inhibiting the phenotypic expression of said resistance mechanism; e.g. *MecA* repressor
3. Multiple resistance mechanism; e.g. Macrolide resistance via modification of the ribosomal binding site and presence of efflux mechanism(s)
4. Level of expression of said resistance mechanism regulated via transcription and translation detectable as the phenotype.

WO 2010/149159 A1 discloses a diagnostic method for identification of MRSA clones using a single one-step PCR. The method is based on the principle that clonal differences within S. *aureus* are reflected in the sequence of a gene (sau1hsdS1) located on the chromosome of this bacterial species. The method includes the use of amplification primers and/or probes for determining the presence of S. *aureus* by detecting said gene. The method further comprises the detection of the gene conferring methicillin resistance (mecA), therefore hallowing discrimination between methicillin-susceptible and methicillin-resistant variants of the clone.

WO 2009/095258 A1 discloses a method for detection of an antibiotic resistance in a micro-organism comprising the steps of exposing suspected micro-organism to a labelled (fluorescent) antibiotic and observing the differences between it and a non-resistant micro-organism of the same type. In particular, the method comprises contacting the labelled antibiotic with a biological sample comprising the micro-organism, detecting the labelled antibiotic in the micro-organism and identifying this micro-organism in the same biological sample, and determining whether the amount of detectable label is altered with respect to the amount of detectable label in the particular micro-organism in its non-resistant form.

Current cultural techniques require the isolation of a discrete colony and the subsequent identification and resistance testing, assuming that a single colony is derived from a single cell and is therefore deemed to be pure. In reality however, the generation of a pure colony from a clinical sample, where pathogens frequently live in bio-film communities, cannon be guaranteed. Equally, using amplification technologies, nucleic acid sequences from multiple cells are extracted and amplified and can therefore render false positive results. Only if identification and resistance can be performed and be read on individual cells, is it possible to a true picture of the invading pathogen.

The increasing spread of antibiotic resistance in both community and healthcare systems necessitates the precision and speed of molecular biology. However, the complexity and cost of these assays prohibits the widespread application in a routine testing environment.

Taking into account the difficulties in identifying a micro-organism and its potential resistance against an antibiotic in a biological sample, it is desirable to be able to quickly identify a pathogen directly from a sample without culturing and without amplification and in addition to be able to detect or exclude the presence of resistance towards an antibiotic of choice.

It is the intention of this invention to provide a solution by enabling the identification of the micro-organism and antibiotic resistance testing on the cellular level. This reduces the complexity of the assays so that an unambiguous assignment of a phenotype can be made for individual cells. The assays are designed to reduce handling and turnaround time to enable screening programmes such as the screening of all incoming patients for e.g. MRSA or/and ORSA.

The present invention exploits the fact that the resistance mechanisms against antibiotics are associated with the presence of specific proteins, specific forms of proteins (e.g. a mutated form of a wild-type protein, or modified proteins), or specific forms of nucleic acids (e.g. a mutated rRNA) within the cell. The present invention provides a method wherein a micro-organism is identified, and, for example at the same time, the antibiotic resistance with this micro-organisms is determined by determination of the presence of resistance factors, for example specific proteins, specific forms of proteins or/and specific forms of nucleic acids which are associated with the antibiotic resistance.

A first subject of the present invention is a method for the simultaneous identification of an individual bacterial cell and detection of an antibiotic resistance in said individual bacterial cell in a biological sample, comprising the steps:
(a) contacting said individual bacterial cell with a first nucleic acid capable of selectively hybridizing with a nucleic acid in said bacterial cell under *in situ* conditions, wherein the first nucleic acid selectively hybridizes with the nucleic acid, and wherein the first nucleic acid is labelled with a first label,
(b) identifying the bacterial cell by the detection of the presence of the first label in said individual bacterial cell,
(c) contacting said same individual bacterial cell with at least one probes for detection of an antibiotic resistance in the bacterial cell under said same *in situ* conditions, wherein said at least one probe is selected from
   (i) antibodies and fragments thereof, the antibodies and said fragments thereof being capable of selectively binding to a resistance factor, and wherein the antibody and said fragments thereof are labelled with a second label,
      wherein said individual bacterial cell is contacted with the antibody or/and said fragment thereof under conditions wherein the antibody or/and the said fragment thereof selectively binds to the resistance factor,
   (ii) second nucleic acids capable of hybridizing with an RNA, coding for a resistance factor, wherein the second nucleic acid is labelled with a third label,
      wherein said individual bacterial cell is contacted with the second nucleic acid under conditions wherein the second nucleic acid selectively hybridizes with the RNA,
   (iii) knottins, cystine-knot proteins or/and aptamers capable of selectively binding to a resistance factor, and wherein the knottins, cystine-knot proteins or/and aptamers are labelled with a fourth label,
      wherein said individual bacterial cell is contacted with the knottin, cystine-knot protein or/and aptamer under conditions wherein the knottin, cystine-kriot protein or/and aptamer selectively binds to the resistance factor, and
   (iv) nitrocefin, wherein said individual bacterial cell is contacted with said nitrocefin under conditions wherein beta-lactamase modifies said nitrofecin,
   and
(d) determining the antibiotic resistance of the bacterial cell by the detection of the presence of one or more of the following labels, the second label, the third label, the fourth label or/and the modified nitrofecin in the individual bacterial cell, wherein steps (b) and (d) are performed simultaneously in said individual bacterial cell.

In the present invention "antibiotic resistance" refers to a resistance of a bacterial cell against an antibiotic when the antibiotic is administered to a subject in need thereof in a dose that is sufficient to successfully eliminate the bacterial cell in its non-resistant form.

The underlying principle of the method is that if an organism is sensitive or resistant to an antibiotic, it will markedly differ from its resistant or sensitive counterpart. The combination of the identification of the bacterial cell and the detection of the antibiotic resistance within one assay, as provided by the present invention, improves the characterisation of bacterial cells in routine diagnostic procedures by increased speed and decreased costs. Furthermore, by fast detection of the antibiotic resistance profile of bacterial cells in a clinical sample, a specific therapy can be initiated at an early stage of infection. Fast identification and characterisation of antibiotic resistance could lead to early isolation of patients, thus leading to a reduction of antibiotic resistances in nosocomial infections. Furthermore, the fast detection of antibiotic resistances in a patient's sample can lead to selection of a specific antibiotic suspected to be active in this patient, resulting in a reduced use of expensive broad-spectrum antibiotics.

On the other hand, patients suspected of being infected with an antibiotic-resistant bacterial cell which could be harmful for other patients (e.g., MRSA or/and ORSA), should be isolated. In some countries, for example in the Netherlands, all patients are isolated upon arrival at a clinic. As soon as they are checked for MRSA or/and ORSA or other problem-causing infections, this isolation can be terminated. Early identification would lead to a shortened isolation phase. The present invention provides a method for detection of an antibiotic resistance which could be performed immediately upon arrival in the clinic. As the results are available within a few minutes, no isolation of patients being negative for MRSA or/and ORSA or other handful infections is required at all, thus reducing costs.

In the present invention, step (a) refers to contacting the individual bacterial cell with a first nucleic acid capable of selectively hybridizing with a nucleic acid in said bacterial cell under *in situ* conditions, wherein the nucleic acid selectively hybridizes with the nucleic acid, and wherein the first nucleic acid is labelled with a first label.

The individual bacterial cell may be included in any sample of biological origin, such as a clinical sample or food sample, suspected of comprising an antibiotic-resistant bacterial cell.

The labelled nucleic acid may in particular be a labelled oligonucleotide, capable of specifically hybridisirig with a nucleic acid in the bacterial cell under in-situ conditions. The labelled oligonucleotide may have a length of up to 50 nucleotides, for example from 10 to 50 nucleotides. The skilled person knows suitable labels. The labelled oligonucleotide may be a linear oligonucleotide. The skilled person knows suitable conditions wherein the nucleic acid selectively hybridizes with the nucleic acid. The labelled oligonucleotide may be a molecular beacon, as for example described in WO 2008/043543 A2. Suitable conditions for hybridisation of molecular beacons are for example described in WO 2008/043543 A2.

The first nucleic acid may comprise at least one sequence selected from RNA, DNA and PNA sequences.

The first nucleic acid may comprise at least one nucleotide analogue, such as a PNA nucleotide analogue.

The first nucleic acid may comprise at least one ribonucleotide and at least one deoxyribonucleotide.

The nucleic acid which has not hybridized with the target sequence may be removed by washing. If the nucleic acid is a molecular beacon, removal can be omitted.

A molecular beacon, as used herein, can be at nucleic acid capable of forming a hybrid with a target nucleic acid sequence and capable of forming a stem-loop structure if no hybrid is formed with the target sequence, said nucleic acid comprising
1. a nucleic acid portion comprising (a1) a sequence complementary to the target nucleic acid sequence, and (a2) a pair of two complementary sequences capable of forming a stem and flanking the sequence (a1),
2. an effector and an inhibitor, wherein the inhibitor inhibits the effector when the nucleic acid forms a stem-loop structure, and wherein the effector is active when the nucleic acid is not forming a stem-loop structure.

The molecular beacon is also termed herein as "beacon", "hairpin", or "hairpin loop", wherein the "open" form (no stem is formed) as well as the "closed" form (the beacon forms a stem) is included. The open form includes a beacon not forming a hybrid with a target sequence and a beacon forming a hybrid with the target sequence. Details of molecular beacons are disclosed in WO 2008/043543 A2.

The molecular beacon may have a length of at least 10, at least 15, at least 16, at least 17, at least 18, at least 19, or a least 20 nucleotides. The molecular beacon may have a length of at the maximum 30, at the maximum 40, or at the maximum 50 nucleotides.

It is preferred that hybridisation is performed at a temperature of between about 25°C and about 65°C, in a more preferred embodiment the temperature is between about 35°C and about 59°C. In an even more preferred embodiment, the temperature is at about 52°C. The incubation time is preferably between about 1 and about 30 minutes. It is more preferred to incubate for up to 15 minutes or for up to 10 minutes, or for about 15 minutes, or for about 10 minutes. After the incubation the carrier may be submerged in 50% ethanol followed by a bath in pure ethanol. Both steps may be run for between about 1 and about 10 minutes. The preferred length of incubation is between about 2 and about 6 minutes. It is more preferred to incubate about 4 minutes. The carrier may then be air-dried (e.g. on a hot plate) and the cells may be embedded in a balanced salt mounting medium.

The biological sample may be fixated or/and perforated before or/and during step (a). Fixation or/and perforation may be performed as described herein. The skilled person knows suitable protocols. Examples of fixation or/and perforation are described, for example in WO 2008/043543 A2.

Step (b) of the method of the present invention refers to identifying the bacterial cell by the detection of the presence of the first label in an individual cell of the bacterium.

"Identification" in the context of the present invention refers to identification of individual microbial cells as belonging to a particular taxonomic category such as species, genus, family, class or/and order, etc. Identification can be performed based on morphological or/and biochemical classifications. The bacterial cell may be selected from the group consisting of Gram positive and Gram negative bacterial. Preferably, the bacterial cell is selected from the group consisting of *Staphylococcus, Enterococcus, Streptococcus* and *Clostridium.* The Gram negative bacterial cell may be selected from Enterobacteriaceae. The Gram negative bacterial cell from the group of Enterobacteriaceae may be selected from *Escherichia coli, Klebsiella spp., Proteus spp., Salmonella spp.,* and *Serratia marcescens.* The Gram negative bacterial cell may also be selected from *Pseudomonas aeruginosa, Acinetobacter spp., Burkholderia spp., Stenotrophomonas* and *Haemophilus influenzae.*

More preferably, the bacterial cell is selected from the group consisting of Methicillin resistant *Staphylococcus,* Oxacillin resistant *Staphylococcus, Vancomycin* resistant *Staphylococcus,* Vancomycin resistant *Enterococcus,* Vancomycin resistant *Clostridium* and high level Aminoglycoside resistant *Enterococci.*

The microroganism is even more preferably selected from the group consisting of *Staphylococcus aureus,* Methicillin Resistant *Staphylococcus aureus* (MRSA), Oxacillin Resistant *Staphylococcus aureus* (ORSA), Vancomycin Resistant *Staphylococcus aureus* (VRSA), Vancomycin Resistant *Staphylococcus* (VRS), Vancomycin Resistant Enterococci (VRE), *Streptococcus pneumoniae,* drug resistant *Streptococcus pneumoniae* (DRSP), and Aminoglycoside resistant Enterococci (HLAR), Vancomycin resistant *Clostridium difficile* (VRCD).

Preferred is identification of the bacterial cell by fluorescence in-situ hybridisation (FISH). An in-situ hybridisation protocol may be applied as laid down in patent application WO 2008/043543 A2.

The first label may be detected by any suitable method known in the art. In particular, the first label may be detected by a detection method as described herein.

In the present invention, step (c) refers to contacting the same individual bacterial cell with at least one probe for detection of an antibiotic resistance in the bacterial cell under said same in-situ conditions.

In the present invention, a "resistance factor" is a cellular components capable of mediating the resistance towards an antibiotic. Such resistance factor may be protein which is modified or altered in a bacterial cell resistant against the antibiotic, compared with the protein in a non-resistant bacterial cell. Examples of proteins being resistance factors include modified or altered target proteins of the antibiotic mediating the antibiotic action, for example a modified PBP2 protein (termed PBP2' or PBP2a) with low affinity for methicillin and many ß-lactam antibiotics, in particular with low affinity for penicillins. Another example relates to vancomycin-resistant Staphylococcus aureus. In non-resistant Staphylococci, vancomycin binds to peptidoglycan precursors and thereby prevents cross-linking of the cell wall peptidoglycan. In Vancomycin-resistant Staphylococci, a D-alanyl-D-lactate ligase (VanA) modifies the D-Ala-D-Ala terminus to D-alanine-D-lactate (D-Ala-D-Lac). Vancomycin-resistant Staphylococci have a reduced capability of binding vancomycin to modified peptidoglycans comprising a D-alanine-D-lactate C-terminus so that vancomycin becomes ineffective.

Other examples of resistance factors refer to proteins capable of binding an antibiotic, thereby inactivating the antibiotic. Such antibiotic-binding proteins can be different from the target of the antibiotic. Binding of the antibiotic to a protein different from the target can result in resistance, as the antibiotic is no more capable of reaching the target.

Other examples of proteins being resistance factors include proteins capable of inactivating the antibiotic by its enzymatic activity (i.e. enzymes), for example a beta-lactamase.

Other examples of proteins being resistance factors include pumps capable of removing an antibiotic from a bacterial cell cell, for example RND transporters.

In the present invention, the term "beta-lactamase" includes carbapenemase and NDM1.

In the present invention, the modification of a resistance factor being a protein may be a mutation, for example a deletion, insertion or amino acid exchange, compared with the unmodified protein present in a form which does not mediate antibiotic resistances. Also included are frameshift mutations.

The method of the present invention may comprise the induction of the antibiotic resistance in the bacterial cell. An antibiotic resistance can be induced by contacting the bacterial cell with a low concentration of an antibiotic, such as cephatoxin or cefoxitin. The antibiotic may be included in a clinical sample buffer, i.e. a buffer for keeping the bacterial cell obtained from a clinical sample. The clinical sample buffer can be prepared so that essentially no cell growth or/and cell division takes place. Contacting with the antibiotic may be performed in step (a), in step (b), in step (c), in step (d) or/and in an additional step of the method of the present invention. Contacting with the antibiotic may be performed in step (a), in step (b) in step (c) or/and in an additional step of the method of the present invention. Contacting with the antibiotic may also be performed in step (a), in step (b) or/and in an additional step of the method of the present invention. The additional step may be introduced between two of the steps of the method of the present invention, for example between steps (a) and (b), or/and between steps (b) and (c), or may be performed before the step (a).

In the present invention, "induction of antibiotic resistance" preferably iridicates the expression of a resistance factor, as defined herein, against an antibiotic in a bacterial cell capable of being resistant against this antibiotic. In the method of the present invention, induction of an antibiotic resistance may include induction of the expression of the resistance factor. "Induction of an antibiotic resistance", as used herein, does preferably not include conditions allowing growth or/and propagation of the bacterial cell. "Induction of an antibiotic resistance", as used herein, does preferably not include conditions under which a non-resistant bacterial cell acquires the genetic modification causing antibiotic resistance.

In particular, PBP2a can be induced, for example by cephatoxin or/and cefoxitin. Preferably, an antibiotic resistance in MRSA or/and ORSA is induced by induction of PBP2a.

The low concentration of the antibiotic employed in the method of the present invention is preferably below the concentration that is sufficient to successfully eliminate the bacterial cell in its non-resistant form.

Examples of targets of antibiotics include rRNA. As described herein, many antibiotics act via binding to rRNA, thereby disturbing protein biosynthsis. In the present invention, the resistance factor may be a modified rRNA having a lower affinity for the antibiotic compared with the affinity of the unmodified rRNA in a non-resistant cell.

In the present invention, modification of a resistance factor being an rRNA may be a mutation, such as insertion, deletion, or/and nucleotide exchange. The mutation may be a point mutation or single nucleotide mutation. In particular, a modified rRNA being a resistance factor may comprise a point mutation. It is preferred to determine the resistance by point mutations in the 23S ribosomal RNA. It is also preferred to determine the resistance by point mutations in the 16S ribosomal RNA. The point mutations at different position in 23S or 16S rRNA induce resistance to a wide array of antibiotics such as macrolides, ketolides, tetracyclines; thiazolantibiotics, lincosamine, chloramphenicol, streptogramin, amecitin, animosycin, sparsoycin and puromycin. Detailed effects of respective point mutations are listed in Table 2. Point mutations at different positions of the 23S or 16S rRNA can generate an iso-phenotype. It would require an array of oligo-nucleotide probes to cover all possibilities. This invention offers a cost effective arid efficient way of detecting antibiotic resistance mediated by rRNA irrespective of the position of the mutation.

In the present invention, the resistance factor may be selected from PBP2a, β-lactamase, efflux transporters, wherein the efflux transporter is preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters.

In the method of the present invention, the antibiotic resistance can be detected by mRNA encoding the resistance factor, if the resistance factor is a protein or polypeptide, as described herein. The mRNA may include a mutation, for example a deletion, insertion or amino acid exchange, compared with the mRNA encoding a polypeptide which is present in a form which does not mediate antibiotic resistance. Also included are frameshift mutations. In particular, the mRNA encodes PBP2a, β-lactamase, efflux transporters, wherein the efflux transporter is preferably selected from the group consisting of ATP-Binding Cassette (ABC) transporters, Major Facilitator Superfamily (MFS) transporters, (Multidrug and Toxic Compound Extrusion (MATE) transporters and Resistance Nodulation Division (RND) transporters.

In the method of the present invention, a resistance against any antibiotic may be detected in an individual bacterial cell. Preferably, the antibiotic is selected from the group consisting of aminoglycosides, carbacephems, carbapenems, cephalosporins, glycopeptides, macrolides, monobactams, penicillins, beta-lactam antibiotics, quinolones, bacitracin, sulfonamides, tetracyclines, streptogramines, chloramphenicol, clindamycin, and lincosamide.

More preferably, the antibiotics are selected from beta-lactam antibiotics, macrolides, lincosamide, and streptogramins. The antibiotic may also be selected from beta-lactam antibiotics. In particular the antibiotic may be selected from penicillins.

In the present invention, beta-lactam antibiotics in particular include carbapenems, cephalosporins, monobactams, and penicillines.

Even more preferably, the antibiotic is selected from the group consisting of Amikacin, Gentamicin, Kanamycin, Neomycin, Netilmicin, Streptomycin, Tobramycin, Loracarbef, Ertapenem, Imipenem, Cilastatin, Meropenem, Cefadroxil, Cefazolin, Cephalexin, Cefaclor, Cefamandole, Cefoxitin, Cefprozil, Cefuroxime, Cefixime, Cefdinir, Cefditoren, Cefoperazone, Cefotaxime, Cefpodoxime, Ceftazidime, Ceftibuten, Ceftizoxime, Ceftriaxone, Cefsulodine, Cefepime, Teicoplanin, Vancomycin, Azithromycin, Clarithromycin, Dirithromycin, Erythromycin, Roxithromycin, Troleandomycin, Aztreonam, Amoxicillin, Ampicillin, Azlocillin, Carbenicillin, Cloxacillin, Dicloxacillin, Flucloxacillin, Mezlocillin, Nafcillin, Penicillin, Piperacillin, Ticarcillin, Bacitracin, Colistin, Polymyxin B, Ciprofloxacin, Enoxacin, Gatifloxacin, Levofloxacin, Lomefloxacin, Moxifloxacin, Norfloxacin, Ofloxacin, Trovafloxacin, Mafenide, Prontosil, Sulfacetamide, Sulfamethizole, Sulfanilimide, Sulfasalazine, Sulfisoxazole, Trimethoprim, Trimethoprim sulfa, Sulfamethoxazole, Co-trimoxazole, Demeclocycline, Doxycycline, Minocycline, Oxytetracycline, Tetracycline, Chloramphenicol, Clindamycin, Ethambutol, Fosfomycin, Furazolidone, Isoniazid, Linezolid, Metronidazole, Mupirocin, Nitrofurantoin, Platensimycin, Pyrazinamide, Quinupristin/Dalfopristin, Rifampin, Spectinomycin, Amphotericin B, Flucanazole, Fluoropyrimidins, Gentamycin, Methicillin, Oxacillin and clavulanic acid.

Most preferably, the antibiotic is selected from Vancomycin, Methicillin, Oxacillin, Clindamycin, Trimethoprim, Trimethoprim sulfa, Gentamycin, and clavulanic acid.

Table 3 indicates resistance mechanisms against commonly known antibiotics in clinically relevant bacterial cells. Specific aspects of the present invention relate to the identification of an individual bacterial cell and the detection of an antibiotic resistance of said same individual bacterial cell, wherein the combination of bacterial cell and antibiotic resistance is selected from the combinations disclosed in Table 3.

In step (c)(l) an antibody or fragment thereof capable of selectively binding to a resistance factor is contacted with the sample under conditions wherein the antibodies selectively binds to the resistance factor. Selective binding is performed under in-situ conditions. The skilled person knows such conditions. Furthermore, the antibody is labelled with a second label. The killed person knows suitable labels. The second label may be any label as described herein.

In the present invention, the antibody includes a primary antibody capable of selectively binding to the resistance factor, and a secondary antibody labelled with the second label, wherein the secondary antibody is capable of selectively binding to the primary antibody. The strategy of primary and secondary antibodies is well-known in the art. By this strategy, the signal detection can be improved.

In the present invention, the antibody, which is capable of selectively binding to a resistance factor may be generated using methods well known in the art. Such antibodies may include, but are not limited to, polyclonal, monoclonal, and chimeric singe chain antibodies.

For the production of antibodies, various hosts including goats, rabbits, rats, mice, humans, and others, may be treated by injection with the resistance factor or any fragment thereof which has immunogenic properties. Depending on the host species, various adjuvants may be used to increase immunological response.

If the resistance factor is a polypeptide or protein, antibodies capable of specifically binding to the polypeptide or protein can be induced by a second polypeptide comprising a fragment of the polypeptide or protein having an amino acid sequence of at least five amino acids, and preferably at least 10 amino acids.

If the resistance factor is a nucleic acid, in particular an rRNA, antibodies capable of specifically binding to the nucleic acid can be induced by a second nucleic acid comprising a fragment of the nucleic acid having a sequence of at least five nucleotides, and preferably at least 10 nucleotides.

Monoclonal antibodies to the proteins may be prepared using any technique that provides for the production of antibody molecules by continuous cell lines in culture. These include, but are not limited to, the hybridoma technique, the human B-cell hybridoma technique, and the EBV-hybridoma technique (Köhler G. and Milstein C. (1975) Nature 256: 495-497; Kozbor D. et al. (1985) J. Immunol. Methods 81: 31-42; Cote R.J. et al., (1983) Proc. Natl. Acad. Sci. 80: 2026-2030; Cole S.P. et al., (1984) Mol Cell Biochem. 62: 109-120).

In addition, techniques developed for the production of 'chimeric antibodies', the splicing of mouse antibody genes to human antibody genes to obtain a molecule with appropriate antigen specificity and biological activity can be used (Morrison S.L. et al., (1984) Proc. Natl. Acad. Sci. 81: 6851-6855; Neuberger M.S. et al (1984) Nature 312: 604-608; Takeda S. et al., (1985) Nature 314: 452-454). Alternatively, techniques described for the production of single chain antibodies may be adapted, using methods known in the art, to produce singe chain antibodies specific for the protein of the invention or a homologous protein. Antibodies with rotated specificity, but of distinct idiotypic composition, may be generated by chain shuffling from random combinatorial immunoglobulin libraries (Kang A.S. et al., (1991) Proc. Natl. Acad. Sci. 88: 11120-11123). Antibodies may also be produced by inducing in vivo production in the lymphocyte population or by screening recombinant immunoglobulin libraries or panels of highly specific blinding reagents as disclosed in the literature (Orlandi R. et al., (1989) Proc. Natl. Acad. Sci. 86: 3833-3837; Winter G. and Milstein C., (1991) Nature 349: 293-299).

In the present invention, an antibody fragment, which is capable of selectively binding to a resistance factor may be generated using methods well known in the art. For example, such antibody fragments include, but are not limited to, the F(ab')₂ fragments which can be produced by pepsin digestion, of the antibody molecule and the Fab fragments which can be generated by reducing the disulfide bridges of F(ab')₂ fragments. Alternatively, Fab expression libraries may be constructed to allow rapid and easy identification of morioclonal Fab fragments with the desired specificity (Huse W.D. et al., (1989) Science 246: 1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between the protein and its specific antibody. A two-site, monoclonal-based immunoassay utilizing monoclonal antibodies reactive to two non-interfering protein epitopes are preferred, but a competitive binding assay may also be employed (Maddox D.E. et al., (1983) J. Exp. Med. 158: 1211-1216).

The labelled antibody not bound to the resistance factor may be removed by a wash step.

In one aspect, the antibody and the second label are coupled to a bead. The bead may have a diameter of about 10 nm to about 400 µm. The bead comprises a plurality of antibody molecules each capable of binding to the target. In step (c), aggregates comprising a bead and an individual bacterial cell may be formed, wherein said individual bacterial cell comprises a resistance factor to which the antibody selectively binds. By this strategy, removal of the antibodies not coupled to a resistance factor is facilitated compared with removal of free antibodies carrying a label.

In a further aspect, the bead comprises a plurality of second label molecules. Beads carrying a plurality of label molecules can be more readily detected, compared with an antibody molecule carrying a label.

In a particular aspect, the bead may be larger than the bacterial cell. In this aspect, the bead may have a diameter of about 50 µm to about 400 µm, or about 100 µm to about 300 µm, or about 150 µm to about 250 µm, or about 200 µm. An aggregate may be formed in step (c), said aggregate comprising more than one bacterial cell and at least one bead.

In a further particular aspect, the bead may be smaller than the bacterial cell. In particular, the bead may be a micro-bead. The bead may have a diameter of up to 100 nm, or up to about 80 nm, or up to about 60 nm, or up to about 50 nm. Preferred bead have a diameter of about 20 nm or about 40 nm. In this aspect, in step (c), a plurality of beads may be coupled to a bacterial cell.

In the method of the present invention, beads having antibodies coupled thereto, as described herein, may in particular be used for the detection of resistance factors located at the surface of the bacterial cell.

In step (c)(II), a second nucleic acid capable of hybridizing with an RNA coding for a resistance factor is contacted with the individual bacterial cell under conditions wherein the nucleic acid selectively hybridizes with the RNA. The RNA coding for a resistance factor in particular is a mRNA. The skilled person knows suitable conditions wherein the nucleic acid selectively hybridizes with the RNA. For example, the labelled nucleic acid may in particular be a labelled oligonucleotide, capable of specifically hybridizing with a nucleic acid in said individual bacterial cell under *in-situ* conditions. The labelled oligonucleotide may have a length of up to 50 nucleotides, for example from 10 to 50 nucleotides. The labelled oligonucleotide may be a linear oligonucleotide. The skilled person knows suitable conditions wherein the nucleic acid selectively hybridizes with the nucleic acid. The labelled oligonucleotide may be a molecular beacon, as disclosed herein. Suitable conditions for selective hybridisation of molecular beacons are for example described in WO 2008/043543 A2.

In situ hybridisation protocols, in particular FISH protocols, are described, for example, in Wilkinson, D.G. (ed.) "In situ Hybridisation. A practical approach", second edition, "The practical approach series 196", Oxford University Press, 1999.

The second nucleic acid may comprise at least one sequence selected from RNA, DNA and PNA sequences.

The second nucleic acid may comprise at least one nucleotide analogue, such as a PNA nucleotide analogue.

The second nucleic acid may comprise at least one ribonucleotide and at least one deoxyribonucleotide.

The nucleic acid which has not hybridized with the target sequence may be removed by washing. If the nucleic acid is a molecular beacon, removal is not necessary.

In step (c)(III), the individual bacterial cell is contacted with the knottin, cystine-knot protein or/and aptamer under conditions wherein the knottin, cystine-knot protein or/and aptamer selectively binds to the resistance factor. The skilled person knows suitable conditions.

A knottin is a small disulfide-rich protein characterized by a "disulfide through disulfide knot". The structure of knottins is for example described in http:/lknottin.cbs.cnrs.fr.

Cystine-knot proteins are proteins providing a cystine-knot signature. The cystine-knot signature corresponds to the cystine-knot well described in the large family of transforming growth factors. The typical cysteine framework in these proteins consists of four cysteine residues with a cysteine spacing of Cys-(X)3-Cys and Cys-X-Cys, important for a ring structure formed by 8 amino acids. Two additional cysteines form a third disulfide bond which penetrates the ring structure, thus forming a cystine-knot. A description of cystine-knot proteins can be found on http://hormone.stanford.edu/cystine-knot.

Aptamers are oligonucleotide or peptide molecules which are designed for specifically binding to a target molecule. The skilled person knows suitable strategies for design or/and selection of aptamers.

The knottin, cystine-knot protein or/and aptamer and the fourth label may be coupled to a bead. The bead may be a bead, as described herein in the context of step (a).

In step (c)(IV), the individual bacterial cell is contacted with nitrocefin which can be modified by beta-lactamase. Step (c)(IV) is performed under in-situ conditions. When cleaved by beta-lactamase, nitrocefin changes colour from yellow to red, which change can be detected in individual bacterial cells by microscopy.

Step (d) of the method of the present invention refers to determination of the antibiotic resistance of the bacterial cell by the detection of the presence of one or more of the following labels, the second label, the third label, the fourth label, or/and the modified nitrocefin in an individual cell of the bacterium. The second, the third or/and the fourth label may be detected by any suitable method known in the art. In particular, the second, the third or/and the fourth label may be detected by a detection method as described herein, for example by a method described for detection of the first label.

The skilled person knows methods for identification of nitrocefin. For example, nitrocefin cleaved by a beta-lactamase can be detected by a colour shift from yellow to red.

The labels of the present invention, namely the first label, the second label, the third or/and the fourth label, may be any detectable label known in the art. It is preferred that the first label can be discriminated from the second label and the third label. The first label, the second label, the third or/and the fourth label may be detected by a method providing a resolution down to the individual cell. In particular, the first label, the second label, the third or/and the fourth label is detected by a method independently selected from epifluorescence microscopy, flow cytometry, laser scanning techniques, time resolved fluorometry, luminescence detection, isotope detection, hyper spectral imaging scanner, Surface Plasmon Resonance and another evanescence based reading technology.

The first, the second, the third or/and the fourth label, as used herein, may be a luminescent labelling group. Many fluorophores suitable as labelling groups in the present invention are available. The labelling group may be selected to fit the filters present in the market. The labelling group may be any suitable labelling group which can be detected in a bacterial cell. Preferably, the labelling group is a fluorescent labelling group. More preferably, the labelling group is selected from fluorescein, Atto=495-NSI, FAM, Atto550, Atto Rho6G, DY520XL, and DY521XL.

The labelling group may be coupled via a spacer. Many spacers are known in the art and may be applied. Using protein chemistry techniques well known in the art many ways of attaching a spacer and subsequently attaching a fluorophore are feasible. In a preferred embodiment cysteine is chosen as its primary amino group may readily be labelled with a fluorophore. Molecules with longer carbon backbones and other reactive groups well known in the art may also be chosen as linker/spacer.

It is preferred to use in the method of the present invention an oligo-nucleotide labelled with a first label being a fluorophore emitting at a predetermined wavelengths range together with a probe labelled with second, third or/and fourth fluorophore emitting at a different wavelengths range, so that the two fluorophores can be discriminated by luminescence detection. For instance, one of the fluorophors, such as Fluorescein, may emit a green signal, and the other fluorophor may emit a red signal.

In the method of the present invention, steps (a) and (c) may be performed simultaneously or separately. Preferably, steps (a) and (c) are performed simultaneously.

In the method of the present invention, steps (b) and (d) are performed simultaneously.

Preferably, the same detection method is employed for both the identification of the individual bacterial cell and the detection of the antibiotic resistance in the same individual bacterial cell. Said detection method may be any detection method as described herein, for example epifluorescence microscopy, flow cytometry, laser scanning devices or another detection method described herein.

It is also preferred that in the method steps (a), (b), (c) and (d) are performed simultaneously.

By simultaneously performing the steps of the present invention, in particular steps (b) and (d), the characterisation of bacterial cells in routine diagnostic procedures can be improved, as described herein. Improvement refers in particular to automatisation.

Preferably, in-situ hybridisation is combined with detection of antibiotic resistance. More preferably, FISH is combined with detection of antibiotic resistance.

In the method of the present invention identification of the bacterial cell is performed under in-situ conditions, in particular by fluorescence in-situ hybridization (FISH). Detection of antibiotic resistance by a nucleic acid, as described herein, is also performed under in-situ conditions, in particular by fluorescence in-situ hybridization. According to the invention identification of the bacterial cell and detection of antibiotic resistance by a nucleic acid, as described herein, are performed under in-situ conditions, in particular by fluorescence in-situ hybridization (FISH).

FISH, as used herein, can include PNA FISH and bbFISH.

In the method of the present invention steps (a), (b), (c) and (d) comprise in-situ conditions, in particular FISH. In-situ hybridisation, in particular FISH, conventionally calls for specific environments for their respective assays of the state of the art. It was therefore surprising that it was possible to
1. prepare the cells for in-situ hybridisation with pores of sufficient size to allow passage of up to 50-mer oligo-nucleotides
2. make membrane proteins accessible for the probe
3. maintain the integrity of both said proteins and ribosomes to allow the specific binding of the probe
4. Find sufficient binding sites to generate a signal visible under an epifluorescence microscope, in particular under uniform conditions.

If step (c) refers to FISH, this step in particular relates to detection of antibiotic resistance by a nucleic acid according to step (c)(II), as described herein.

The method of the present invention may comprise steps to remove labelled probes which are not bound to a bacterial cell. Such steps may improve the signal-to-noise ratio.

The method steps (a), (b), (c) and (d) can be performed on an automated platform. In particular, simultaneous detection of the first label and the second, the third, or/and the fourth label can be performed on an automated platform by the simultaneous or consecutive detection of the signals from the first label and the second, the third, or/and the fourth label. Detection of the signals can be performed by computer analysis of one or more microscopic images. Simultaneous detection of the first label and the second, the third, or/and the fourth label is preferred. The first label and the second, the third, or/and the fourth label are preferably different.

In the present invention, the bacterial cell in its non-resistant form can be employed as a reference to determine the presence of the first label, the second label, the third label, the fourth label, or/and the modified nitrocefin. The bacterial cell in its non-resistant form may be added to the sample, or may be presented in a separate preparation. The bacterial cell in its non-resistant form may carry at least one further label. Any label as described herein may be employed, provided this label is suitable for discrimination from the label employed for detection of the bacterial cell or/and for identification of the antibiotic resistance, or/and other bacterial cells present in the assay of the present invention. The amount of detectable label in a bacterial cell in its non-resistant form may also be provided in the form of specific values or ranges of the amount of detectable label for one or more combinations comprising (a) the bacterial cell, (b) an antibiotic, and (c) at least one labelling group, for instance in the form of a data sheet. In particular, a kit of the present invention may comprise said bacterial cell in its non-resistant form or/and said data sheet.

The method of the present invention may also employ the bacterial cell in its resistant form as a further control, or specific values or ranges of the amount of detectable label in a bacterial cell in its resistant form for one or more combinations comprising (a) the bacterial cell, (b) an antibiotic, and (c) at least one labelling group, for instance in the form of a data sheet, as described above.

The bacterial cell may be detected or/and identified by an increase of the amount of detectable label of at least 5%, at least 10%, at least 20%, at least 30%, at least 40%, at least 50%, at least 60%, at least 70%, at least 80%, at least 90%, at least 100%, at least 150%, or at least 200% with respect to the amount of detectable label in the bacterial cell in its non-resistant form.

In the method of the present invention, the resistance of beta-lactam antibiotics can be detected by a modified or altered surface receptors or/and glycopeptides. For example, a MRSA or/and ORSA having a modified Penicillin Binding Protein (PBP2a) can be detected. In another examples, a VRE, VRSA or VRCD comprising modified peptidoglycans comprising a D-alanine-D-lactate C-terminus can be detected.

In the method of the present invention, the resistance towards beta-lactam antibiotics can be detected by a mRNA. For example, a MRSA or/and ORSA expressing a mecA RNA can be detected, which for, example, is not present in Methicillin Sensitive *Staphylococcus aureus* (MSSA). In another example, a VRE, VRSA or VRCD comprising a modified vanA, vanB or/and vanC mRNA can be detected.

A preferred resistance factor is the PBP2 protein (Penicillin Binding Protein) in *Staphylococcus* encoded by the *mecA* gene. In *Staphylococcus* resistant against beta-lactam antibiotics, the *mecA* gene encodes a modified PBP2 protein (PBP2' or PBP2a) with low affinity for methicillin and many ß-lactam antibiotics, in particular with low affinity for penicillins. Thus, in a more preferred embodiment, (a) the bacterial cell is a MRSA or/and ORSA strain harbouring the *mecA* gene, which encodes a modified PBP2 protein (PBP2' or PBP2a) with low affinity for methicillin and many ß-lactam antibiotics, in particular with low affinity for penicillins, and (b) the antibiotic is a beta-lactam antibiotic.

In the method of the present invention, the resistance towards beta-lactam antibiotics can be detected by anti-beta-lactamase antibodies including antibodies against carbapenemases or/and NDM1. In particular, the resistance towards penicillins can be detected by anti-beta-lactamase antibodies.

In the method of the present invention, the resistance towards beta-tactam antibiotics, in particular penicillins, can be detected by compounds which are cleaved by a beta-lactamase. For example, nitrocefin changes colour from yellow to red when cleaved by a beta-lactamase.

In one particular aspect, in the method of the present invention, the resistance against a beta-lactam antibiotic, such as a penicillin, is detected by an antibody or/and a fragment thereof, specifically binding to beta-lactamase or/and PBP2a binding protein.

In yet another particular aspect, in the method of the present invention, the resistance against a beta-lactam antibiotic, such as a penicillin, is detected by a nucleic acid specifically hybridizing with an RNA encoding for beta-lactamase or/and PBP2a binding protein.

In yet another particular aspect, in the method of the present invention, the resistance against a beta-lactam antibiotic is detected by a knottin, a cystine knot protein or/and an aptamer specifically binding to beta-lactamase or/and PBP2a binding protein.

In yet another particular aspect, in the method of the present invention, the resistance against a beta-lactam antibiotic is detected by nitrocefin modified by beta-lactamase.

In the method of the present invention, the resistance towards quinolones, macrolides, ketolides, aminoglycosides or/and lincosamides can be detected by a labelled antibody directed against an efflux pump, in particular an efflux pump as described herein.

In the method of the present invention, the resistance towards quinolones, macrolides, ketolides, aminoglycosides or/and lincosamides can be detected by a mutation in mRNA, as described herein.

In the method of the present invention, the resistance towards quinolones, macrolides, ketolides, aminoglycosides or/and lincosamides can be detected by a mutation in rRNA, in particular mutations of 23S rRNA or 16S RNA, as described herein.

In yet another particular aspect, in the method of the present invention, (a) the bacterial cell is a Methicillin Resistant *Staphylococcus aureus* (MRSA) or/and an Oxacillin Resistant *Staphylococcus aureus* (ORSA), and (b) the antibiotic resistance is detected by the expression of a modified or altered Penicillin Binding Protein 2 (termed PBP2a). In this aspect MRSA or/and ORSA is discriminated from other Staphylococci, some of which can also express PBP2a. Preferably, the MRSA or/and ORSA is discriminated from *Staphylococcus epidermidis,* which can constitutively express PBP2a. In this aspect, the expression of PBP2a can be induced, as described herein.

In yet another particular aspect, in the method of the present invention, the bacterial cell is a Methicillin Resistant *Staphylococcus aureus* (MRSA) or/and an Oxacillin Resistant *Staphylococcus aureus* (ORSA), and wherein the antibiotic resistance is detected by the expression of a mecA protein.

In yet another particular aspect, in the method of the present invention, the bacterial cell is a Methicillin Resistant *Staphylococcus aureus* (MRSA) or/and an Oxacillin Resistant *Staphylococcus aureus* (ORSA), wherein the Methicillin Resistant *Staphylococcus aureus* (MRSA) or/and the Oxacillin Resistant *Staphylococcus aureus* (ORSA) are discriminated from a Methicillin Sensitive *Staphylococcus aureus* (MSSA) by the detection of the expression of a mecA protein.

In yet another particular aspect, in the method of the present invention, the bacterial cell is selected from Vancomycin Resistant *Staphylococcus aureus* (VRSA), Vancomycin Resistant Staphylococcus (VRS), Vancomycin Resistant Enterococci (VRE) and Vancomycin Resistant Clostridium difficile (VRCD), and wherein the antibiotic resistance is detected by the expression of a peptide selected from vanA protein, vanB protein, vanC protein or/and modified peptidoglycans comprising a D-alanine-D-lactate C-terminus.

In yet another particular aspect, in the method of the present invention, (a) the bacterial cell is selected from Gram negative bacteria, as described herein, and (b) a resistance against beta lactam antibiotics is detected.

In yet another particular aspect, in the method of the present invention, (a) the bacterial cell is selected from Streptococci, and (b) a resistance to macrolides, lincosamide and streptogramin (MLS) is detected.

In yet another particular aspect, in the method of the present invention, (a) the bacterial cell is drug resistant Streptococcus pneumoniae (DRSP), and (b) a resistance to towards beta-lactam antibiotics and macrolides is detected.

In yet another particular aspect, in the method of the present invention, high level Aminoglycoside resistant Enterococci (HLAR) are detected.

The biological sample comprising the individual bacterial cells may be pretreated in order to facilitate binding of the labelled antibiotic and identification of the bacterial cell.

The biological sample may be heat-fixed on a carrier (for example on a slide) according to their designated labelled probes according to step (a) and (c), as described herein, for instance at about 45°C to about 65°C, preferably at about 50°C to about 55°C, more preferably at about 52°C.

If the bacterial cell is a Gram positive bacterium, it may be perforated by a suitable buffer. Gram positive cells may be perforated with a bacteriocin or/and a detergent. In a preferred embodiment a biological detergent is employed, and a specially preferred embodiment Nisin is combined with Saponin. In addition lytic enzymes such as Lysozyme and Lysostaphin may be applied. Lytic enzymes may be balanced into the equation. If the sample is treated with ethanol, the concentration of the active ingredients may be balanced with respect to their subsequent treatment in ethanol. In a more preferred embodiment the concentration of Lysozyme, Lysostaphin, Nisin and Saponin is balanced to cover all Gram positive organisms. An example of a Gram Positive Perforation Buffer is given in Table 1. It is contemplated that variations of the amounts and concentrations, and application temperatures and incubation times are within the skill in the art.

In yet another preferred embodiment, the method of the present invention is a diagnostic method. As described herein, the method of the present invention detects an individual bacterial cell in a biological sample.

Another subject of the present invention relates to the use of a kit suitable for simultaneously identifying an individual bacterial cell and detecting an antibiotic resistance in said individual bacterial cell in a biological sample, wherein said kit is used in the method of any one of claims 14 to 20, said use comprising
(a) the use of a first nucleic acid capable of selectively hybridizing with a nucleic acid in said individual bacterial cell under *in situ* conditions, wherein the first nucleic acid is labelled with a first label, and
(b) the use of at least one probe for detection of an antibiotic resistance in said individual bacterial cell under said same *in situ* conditions, wherein said at least one probe is selected from
   (i) antibodies and fragments thereof, wherein the antibodies and fragments thereof are capable of selectively binding to a resistance factor, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters, and wherein the antibody and said fragments thereof are labelled with a second label,
   (ii) second nucleic acids capable of hybridizing with an RNA coding for a resistance factor, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters, and wherein the second nucleic acid is labelled with a third label,
   (iii) knottins, cystine-knot proteins or/and aptamers capable of selectively binding to a resistance factor, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters, and wherein the knottins, cystine-knot proteins or/and aptamers are labelled with a fourth label, and
   (iv) nitrocefin which can be modified by beta-lactamase.

The kit of the present invention is used in the method of the present invention. The components (a) or/and (b) of the kit may be components as described herein in the context of the method of the present invention.

As indicated herein, the kit may comprise further components, such as a data sheet providing information about the amount of detectable label in at least one combination of bacterial cell, antibiotic and label, or a sample of a bacterial cell in its non-resistant or/and resistant form, e.g. for control purposes.

The kit is used in the detection of an antibiotic resistance in an individual bacterial cell in particular in a biological sample.

The present invention is further illustrated by the following tables.
**Table 1** describes the composition of perforation buffers employed in the present invention.
**Table 2:** Antibiotic resistance due to mutations on the 23S rRNA.
**Table 3:** Antibiotic resistance mechanism in clinically relevant microorganisms and alteration in the amount of antibiotics in resistant bacterial cells. The alteration of the amount of detectable labelled antibiotics in bacterial cells in their resistant form is determined relative to its non-resistant form. The amount is expressed in % change of fluorescence (decrease and increase, respectively) of an antibiotic which carries a fluorescent label.

### References

1. Chambers, H. F. (1997). Methicillin resistance in staphylococci: molecular and biochemical basis and clinical implications. Clinical Microbiology Reviews 10, 781-791.
2. Swenson, J. M., Williams, P., Killgore, G. et al. (2001). Performance of eight methods, including two new methods for detection of oxacillin resistance in a challenge set of Staphylococcus aureus organisms. Journal of Clinical Microbiology 39, 3785-8
3. Van Leeuwen, W. B., Van Pelt, C., Luijendijk, A. et al. (1999). Rapid detection of methicillin resistance in Staphylococcus aureus isolates by the MRSA-screen latex agglutination test. Journal of Clinical Microbiology 37, 3029-30.
4. Louie, L., Majury, A., Goodfellow, J. et al. (2001). Evaluation of a latex agglutination test (MRSA-Screen) for detection of oxacillin resistance in coagulase-negative staphylococci. Journal of Clinical Microbiology 39, 4149-51.
5. Skov, R., Smyth, R., Clausen, M. et al. (2003). Evaluation of cefoxitin 30 µg disc on Iso-Sensitest agar for detection of methicillin-resistant Staphylococcus aureus. Journal of Antimicrobial Chemotherapy 52, 204-7
6. Felten, A., Grandry, B., Lagrange, P. H. et al. (2002). Evaluation of three techniques for detection of low-level methicillin-resistant S. aureus (MRSA): a disc diffusion method with cefoxitin and moxalactam, the Vitek 2 system, and the MRSA-screen latex agglutination test. Journal of Clinical Microbiology 40, 2766-71.
7. Cauwelier, B., Gordts, B., Descheemaecker, P. et al. (2004). Evaluation of a disk diffusion method with cefoxitin (30 µg) for detection of methicillin-resistant Staphylococcus aureus. European Journal of Clinical Microbiology and Infectious Diseases 23, 389-92.
8. Kluytmans, J., Van Griethuysen, A., Willemse, P. et al. (2002). Performance of CHROM agar selective medium and oxacillin resistance screening agar base for identifying Staphylococcus aureus and detecting methicillin resistance. Journal of Clinical Microbiology 40, 2480-2.
9. Louie, L., Matsumura, S. O., Choi, E. et al. (2000). Evaluation of three rapid methods for detection of methicillin resistance in S. aureus. Journal of Clinical Microbiology 38, 2170-3.
10. National Committee for Clinical Laboratory Standards. (2003). Methods for Dilution Antimicrobial Susceptibility Tests for Bacteria that Grow Aerobically: Approved Standards M7-A6 and M100-S13. NCCLS, Wayne, PA, USA.
11. WO 2010/149159 A1
12. WO 2009/095258 A1
13. WO 2008/043543 A2

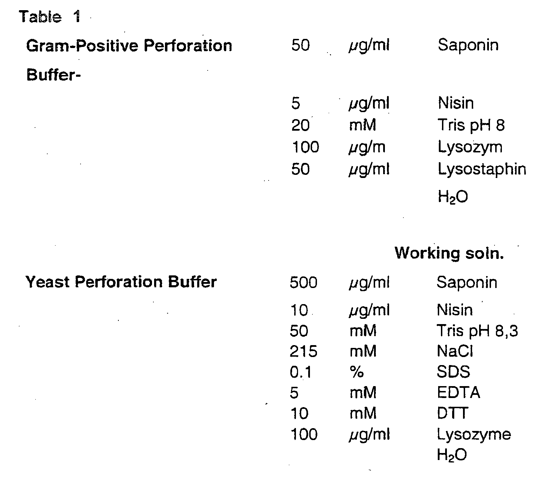

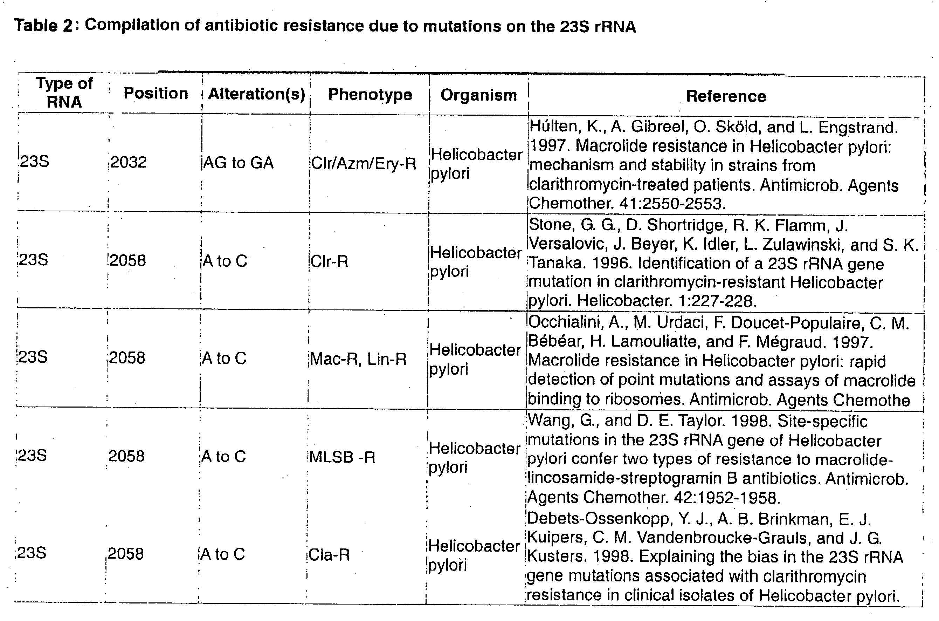

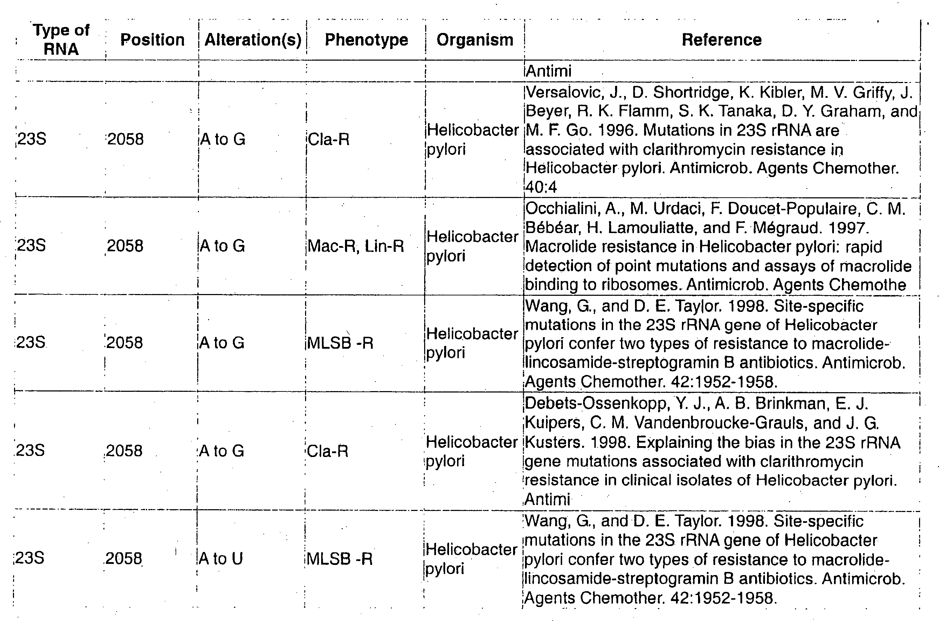

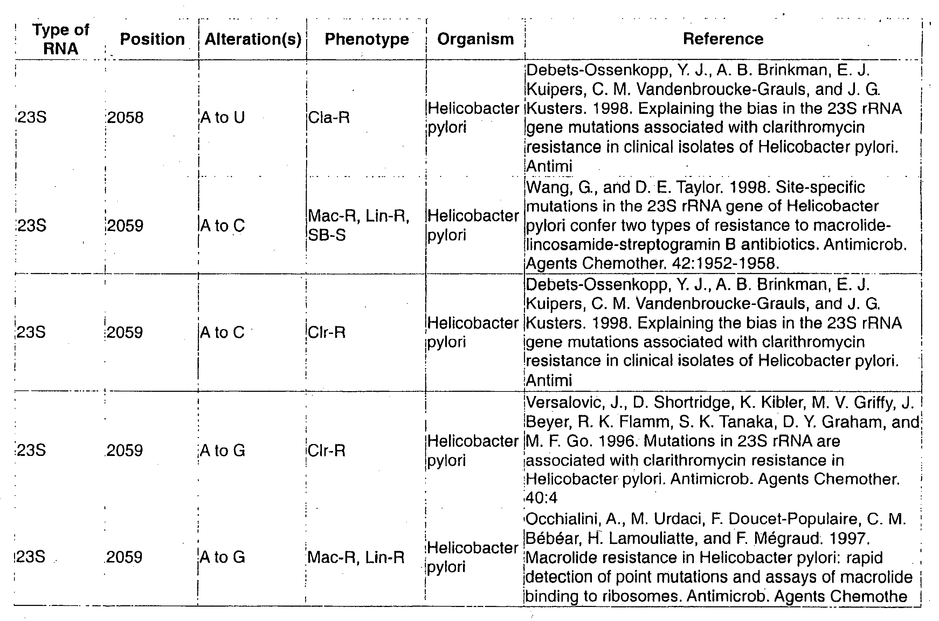

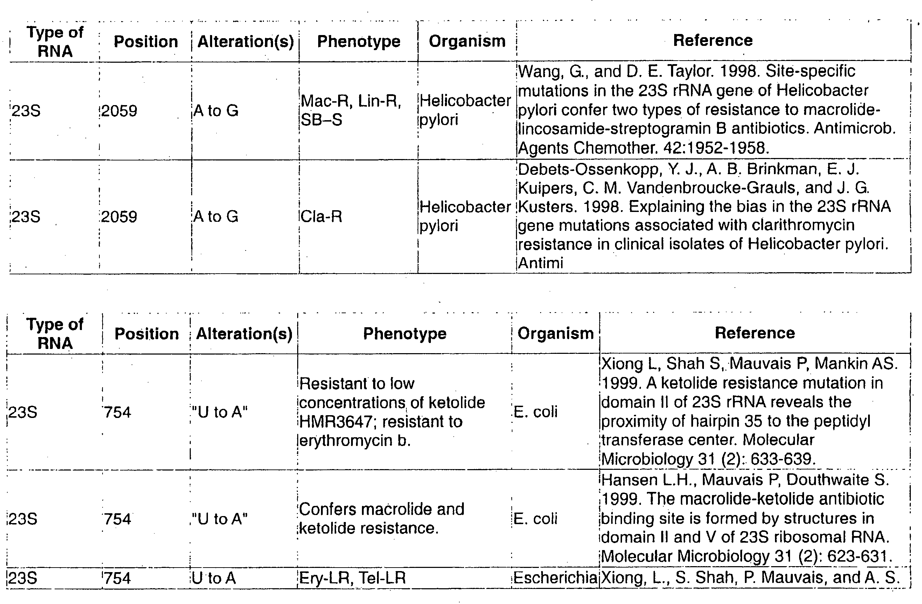

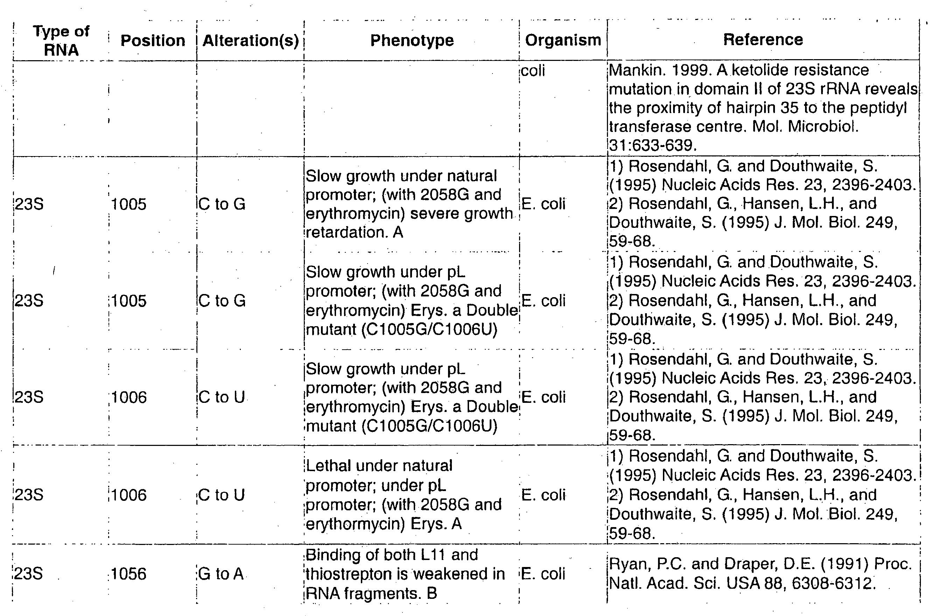

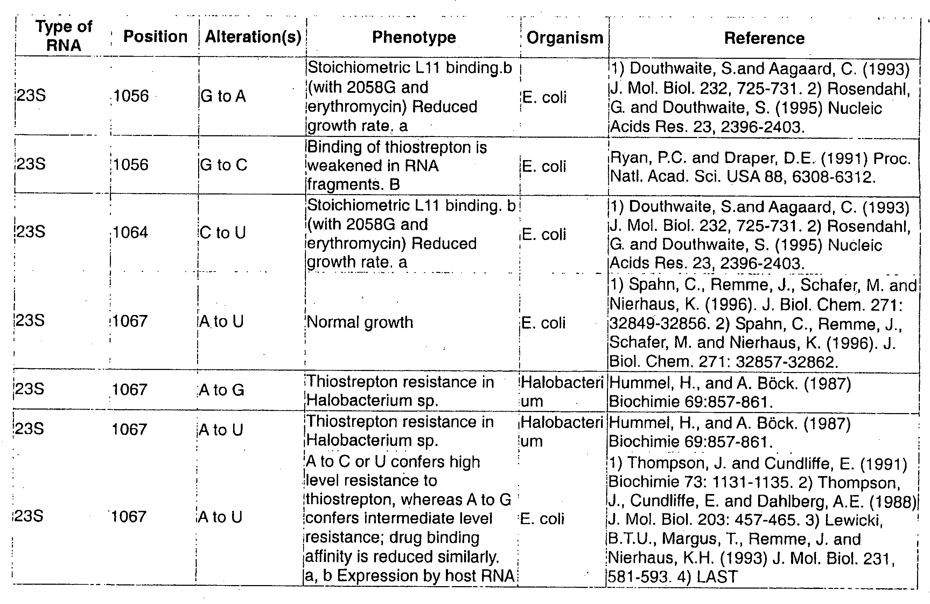

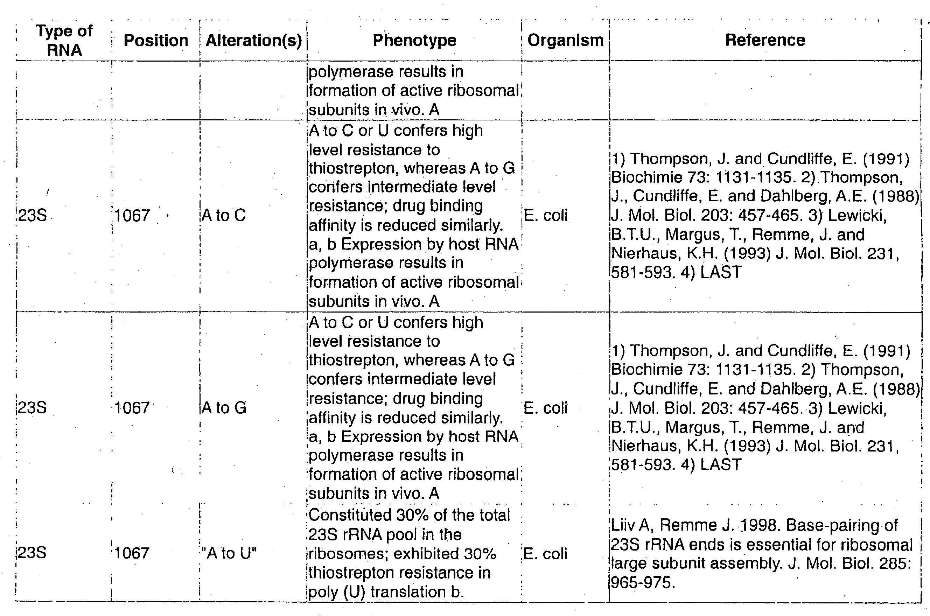

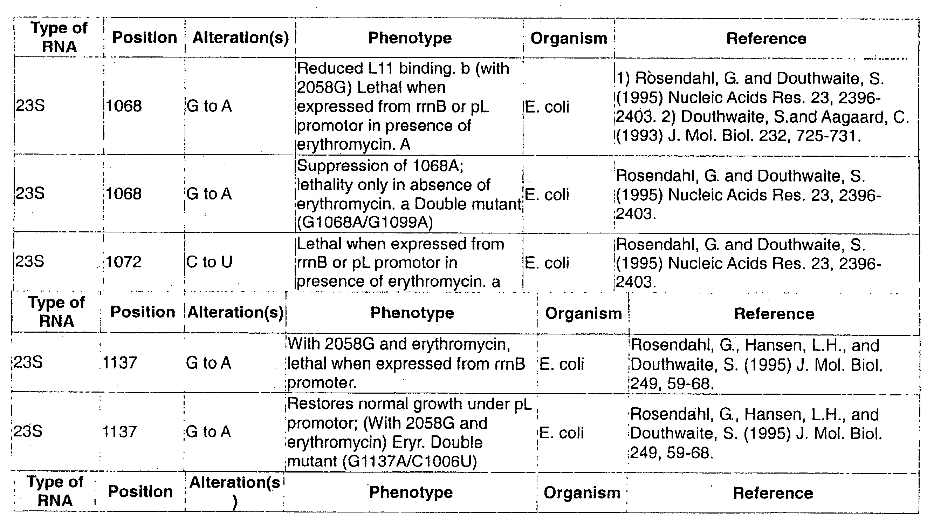

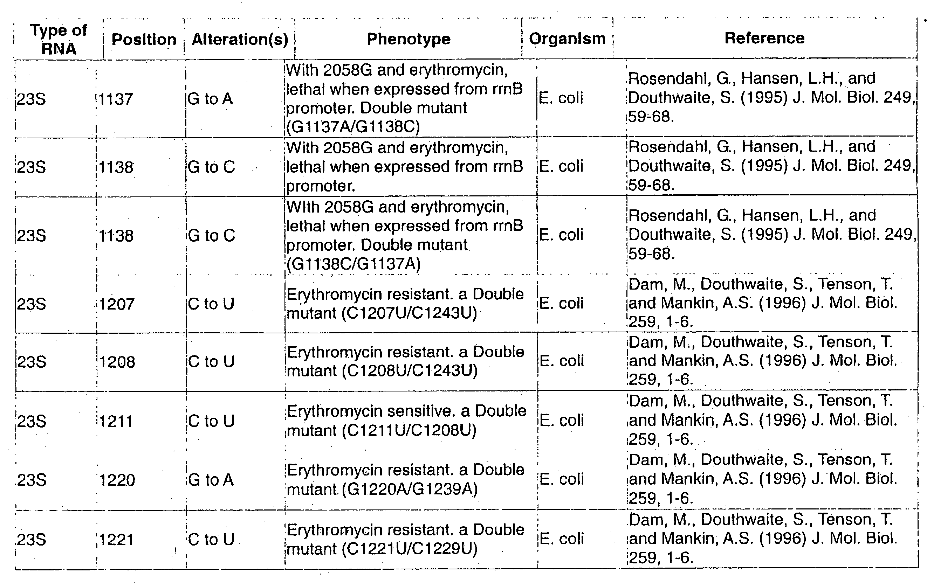

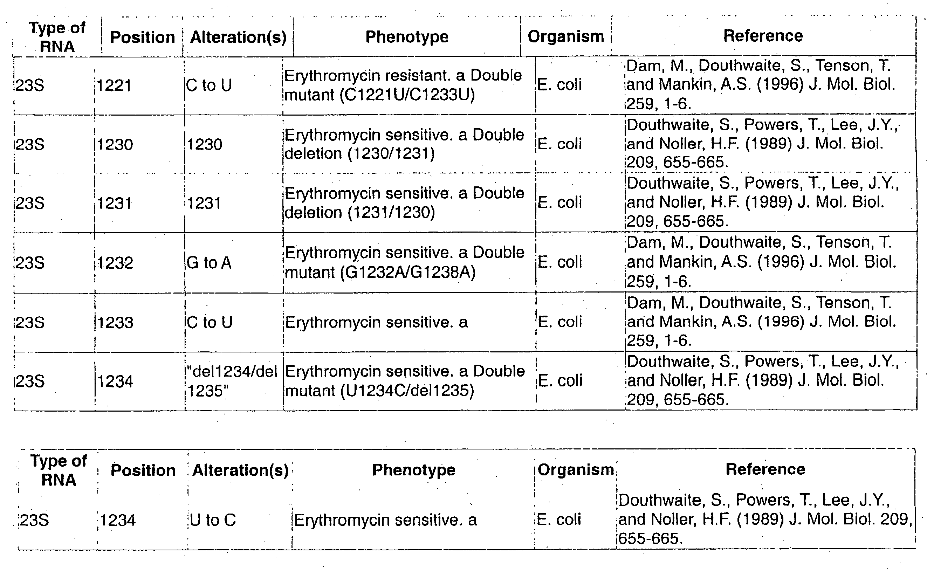

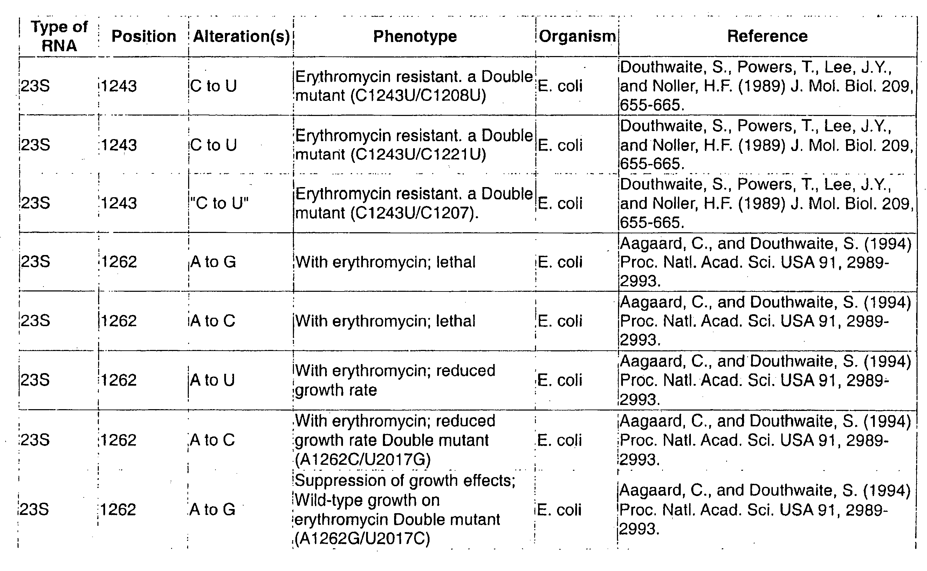

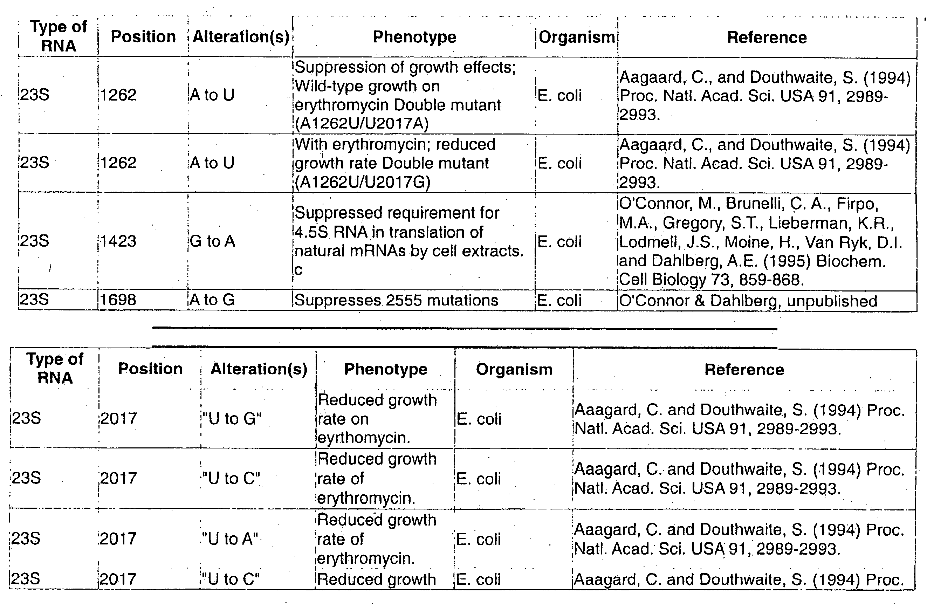

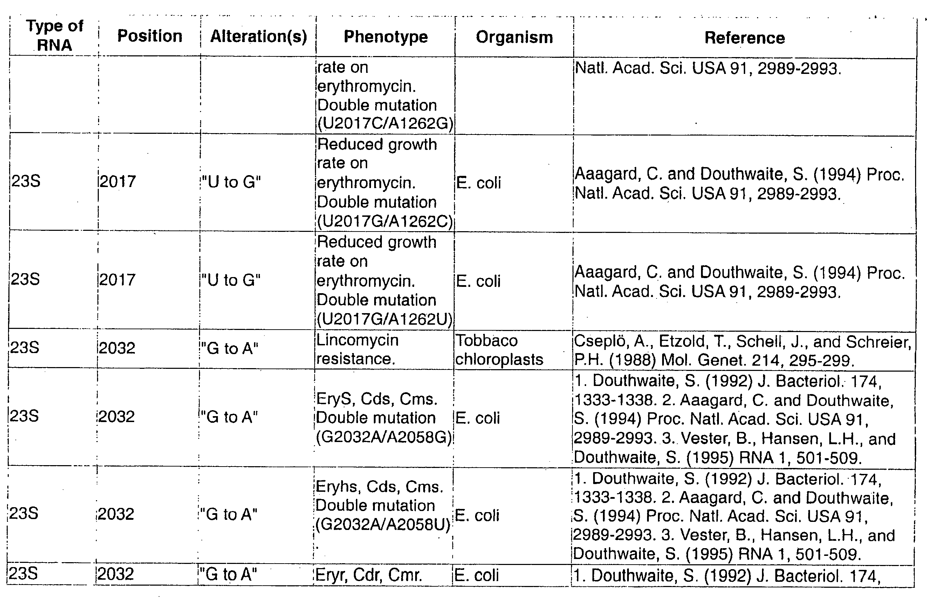

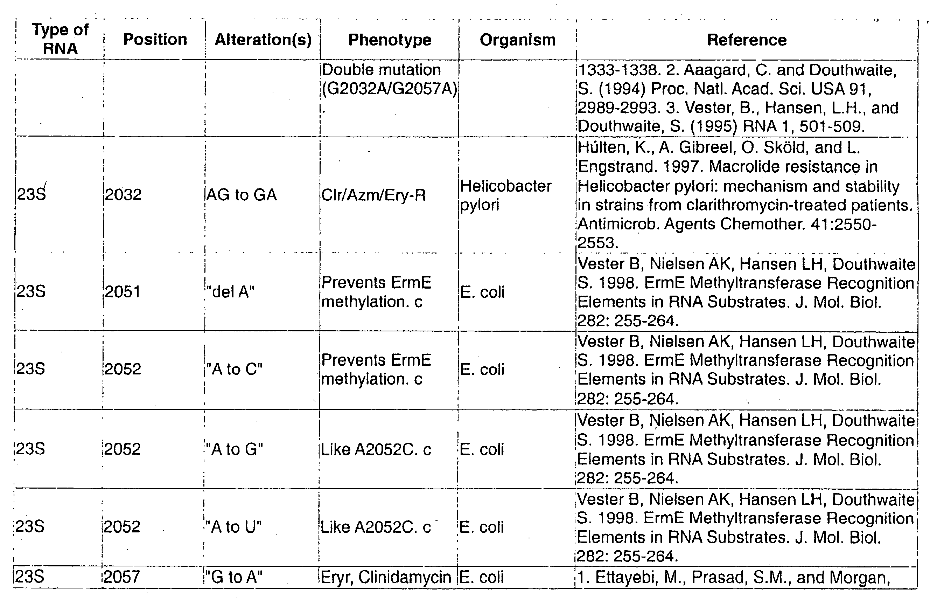

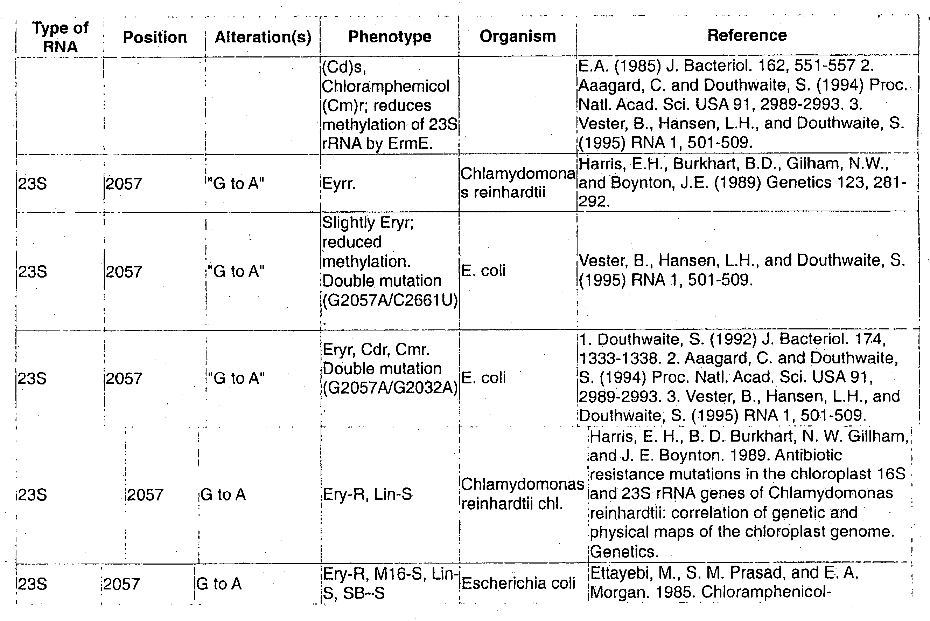

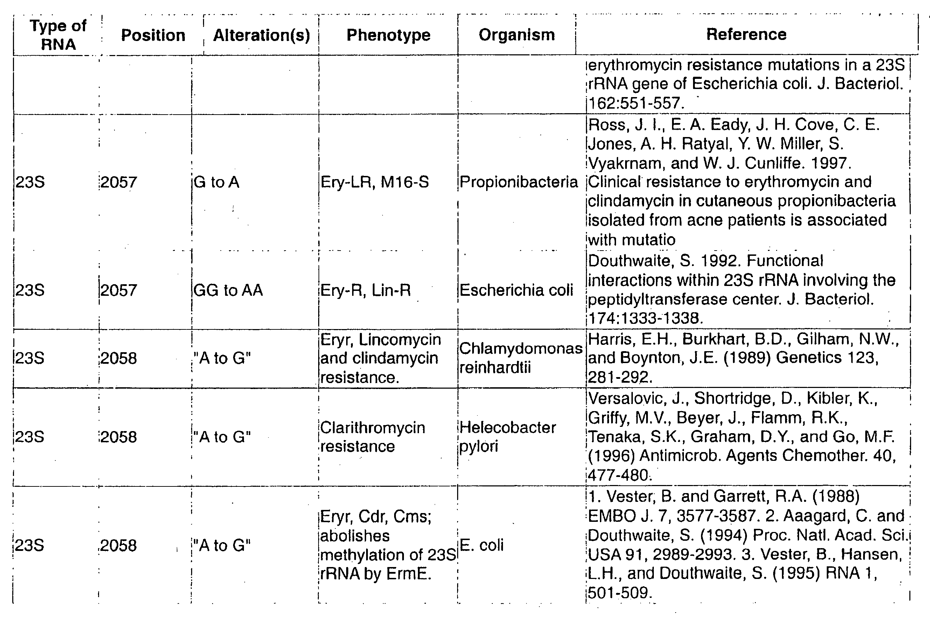

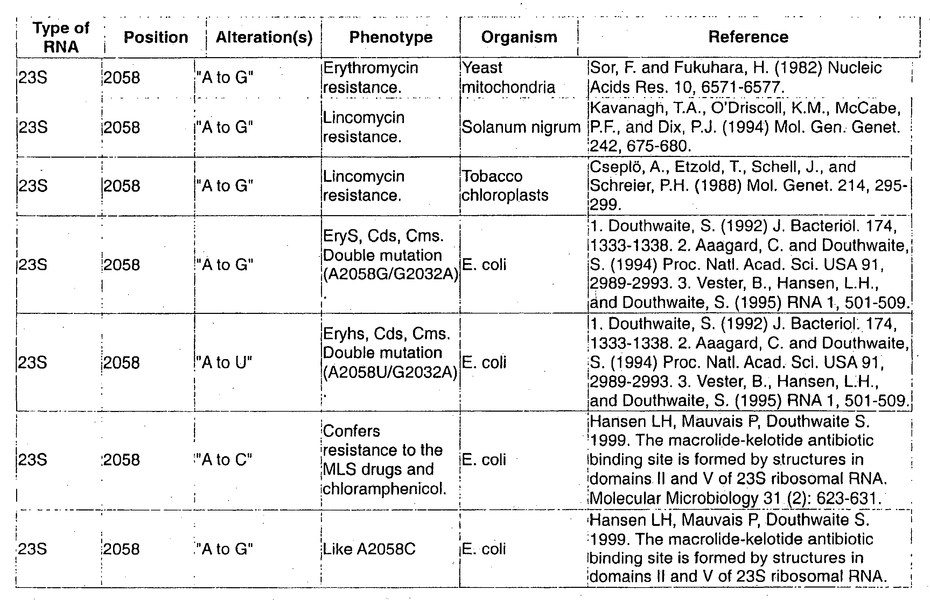

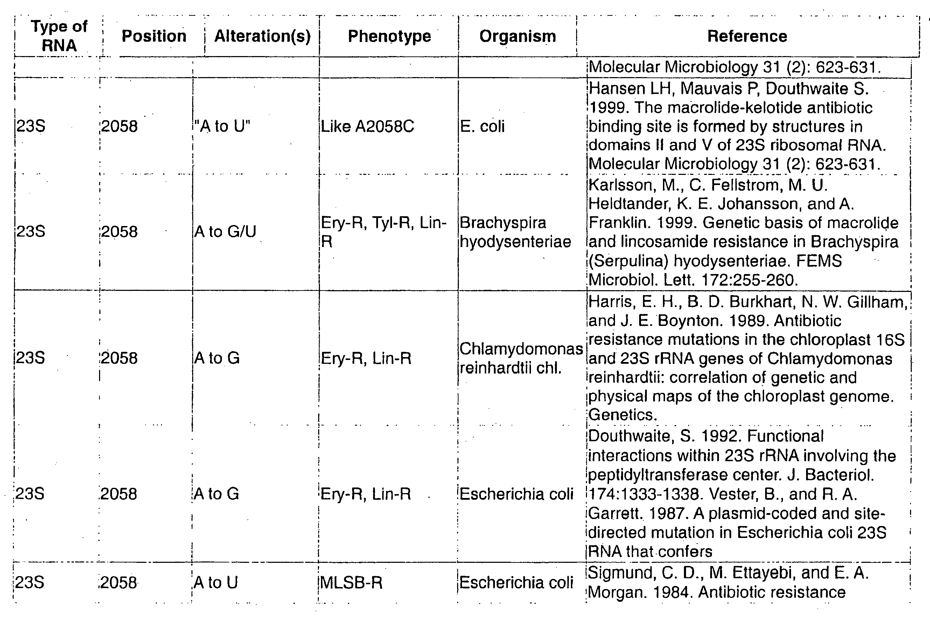

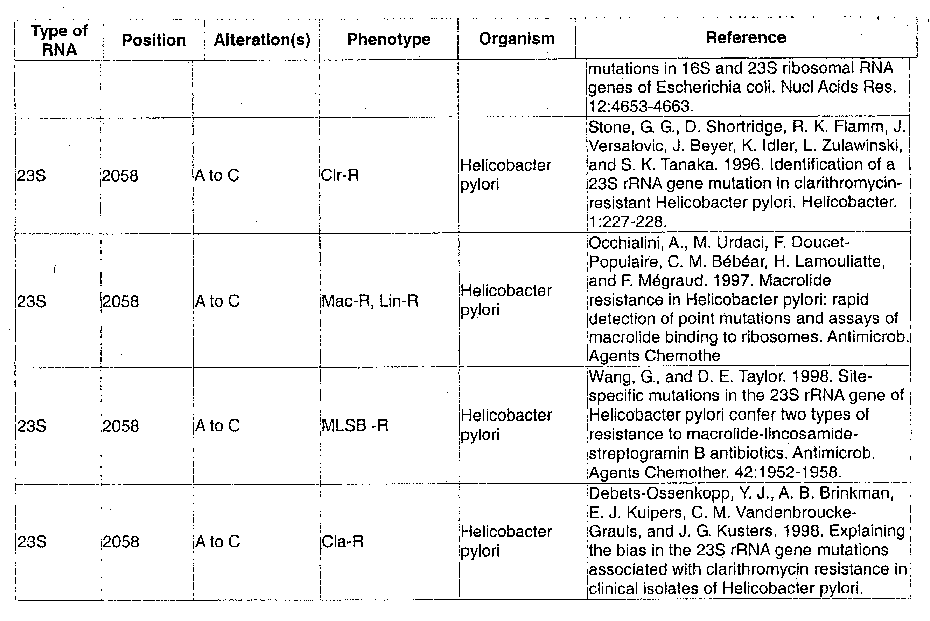

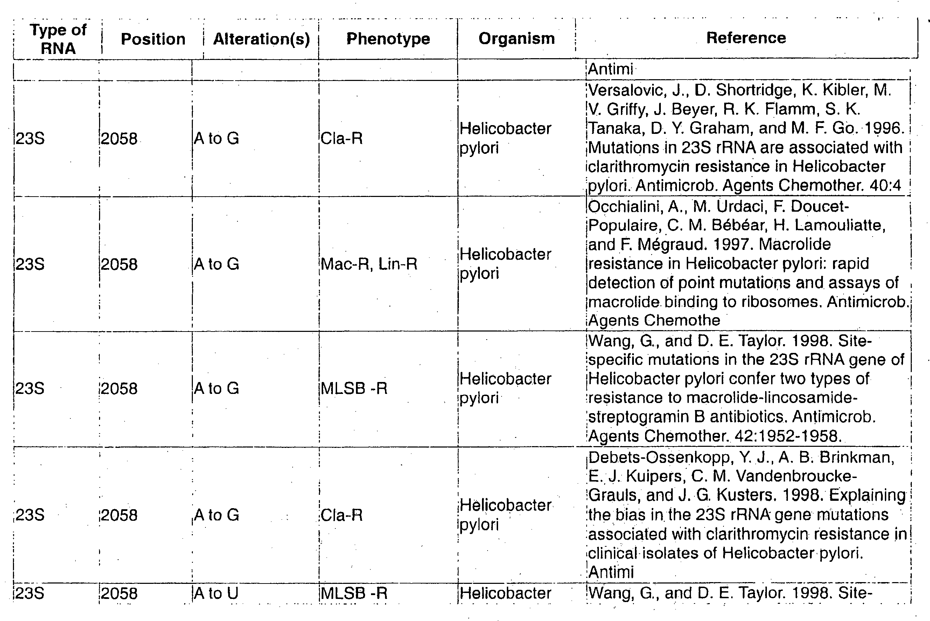

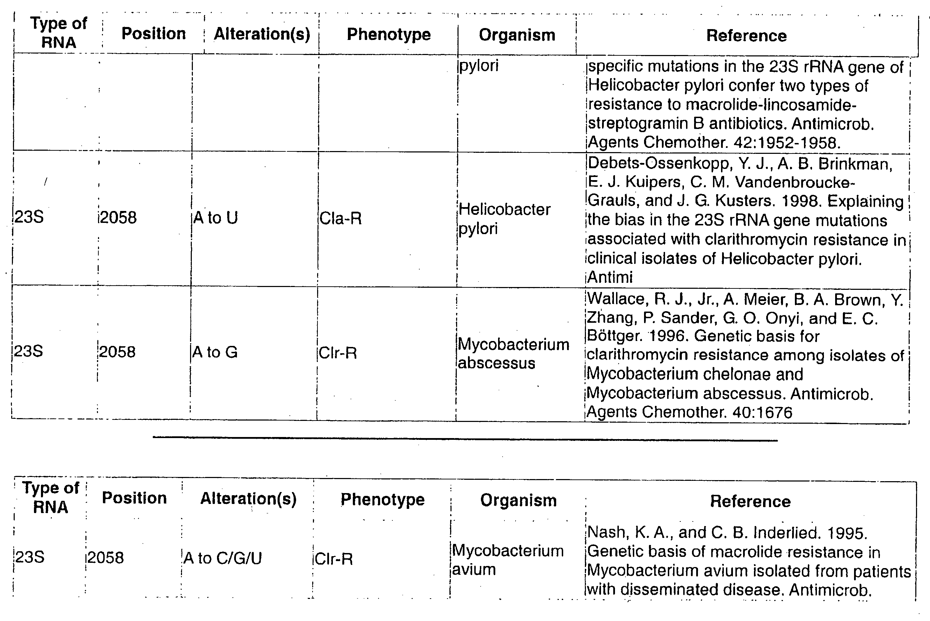

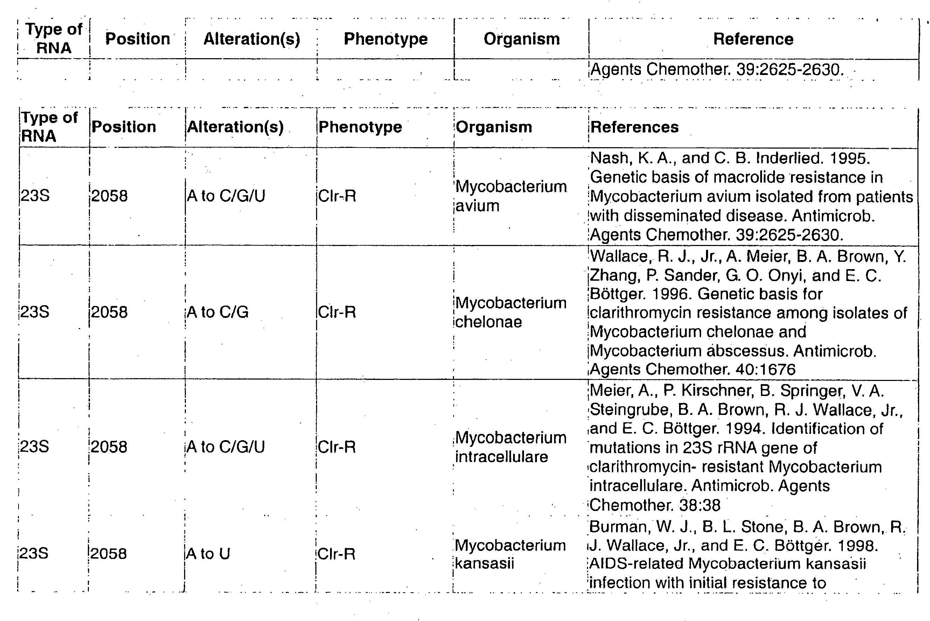

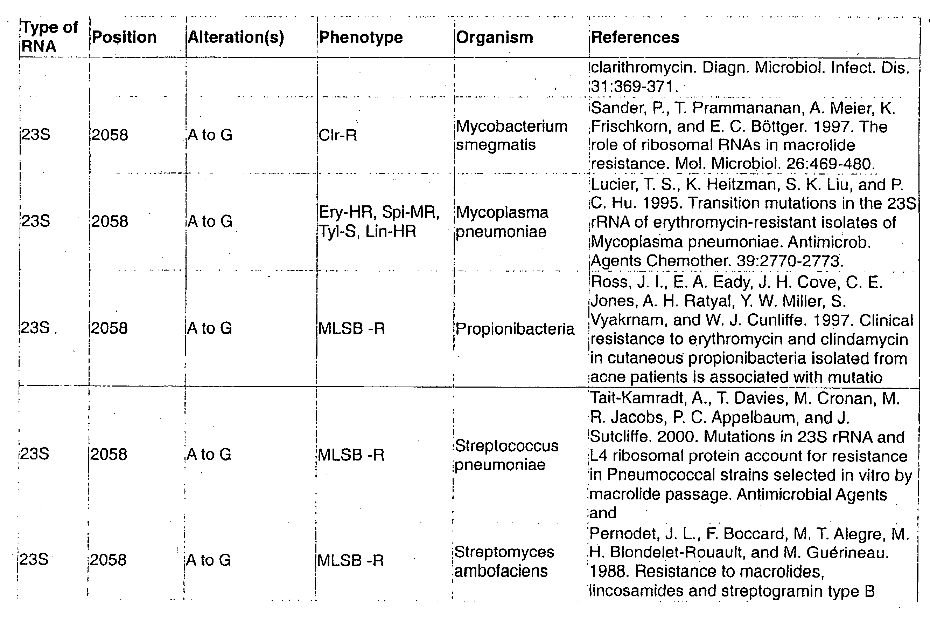

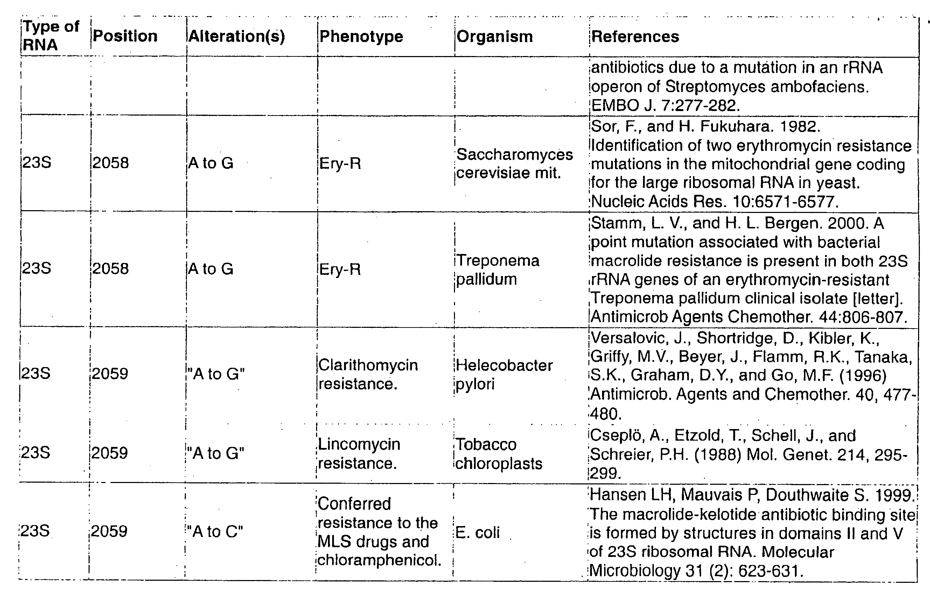

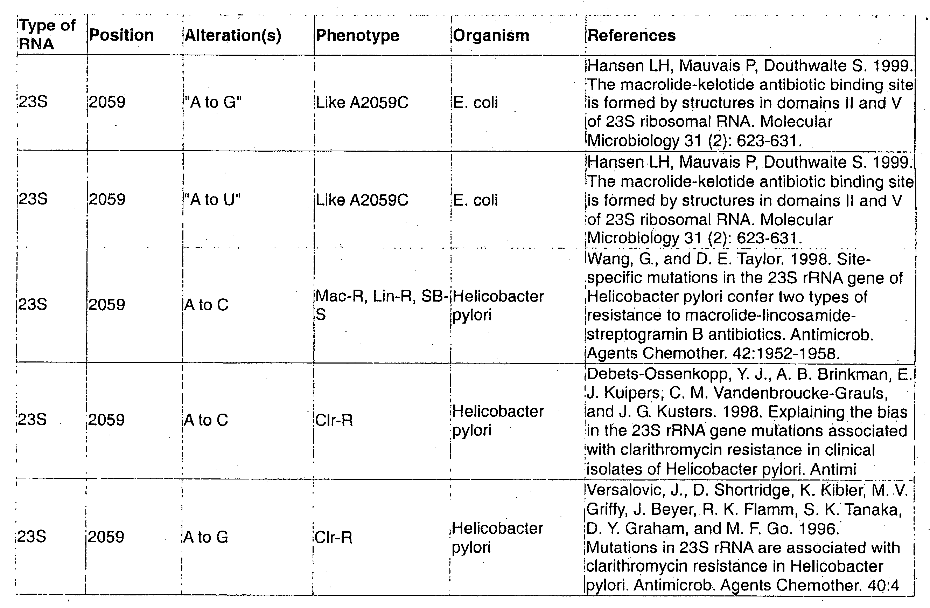

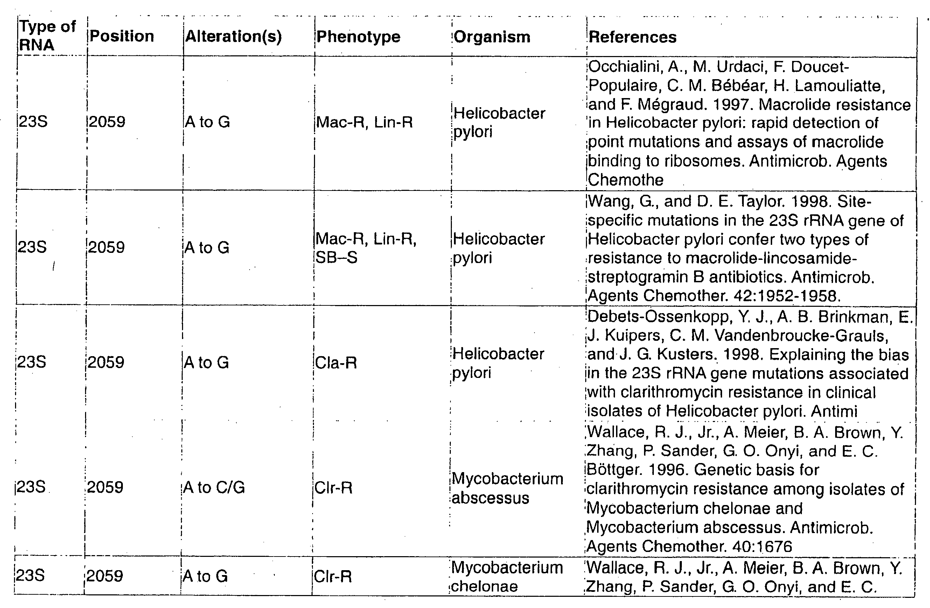

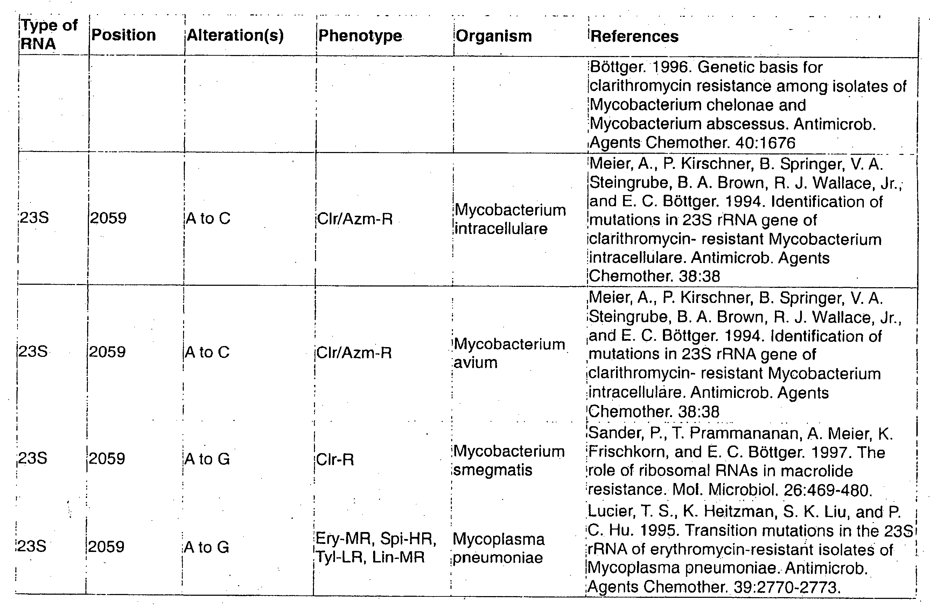

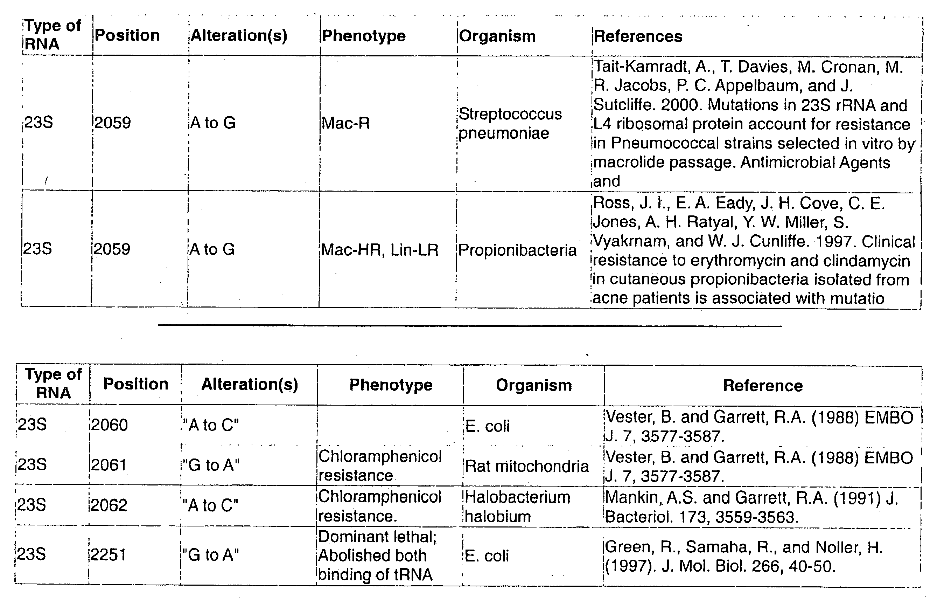

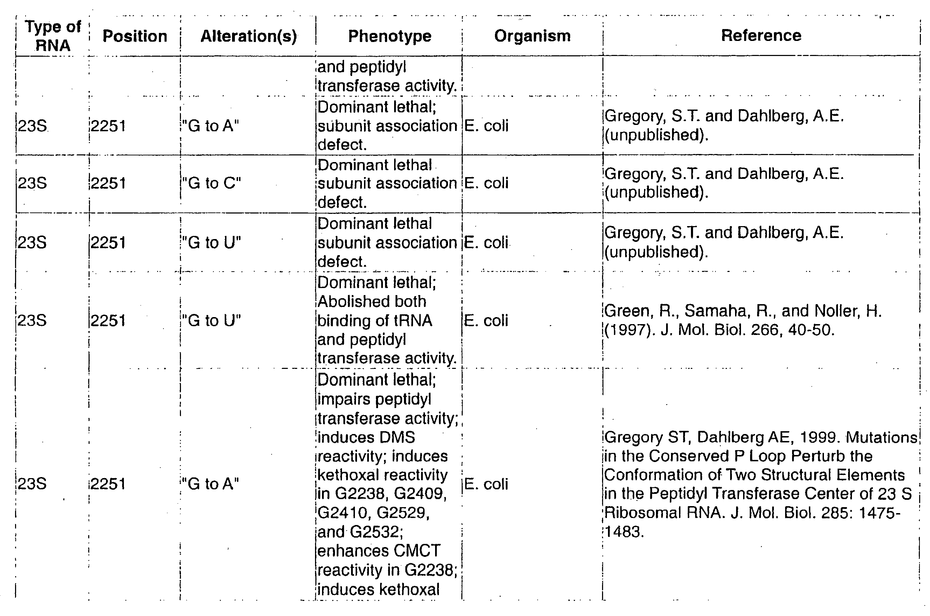

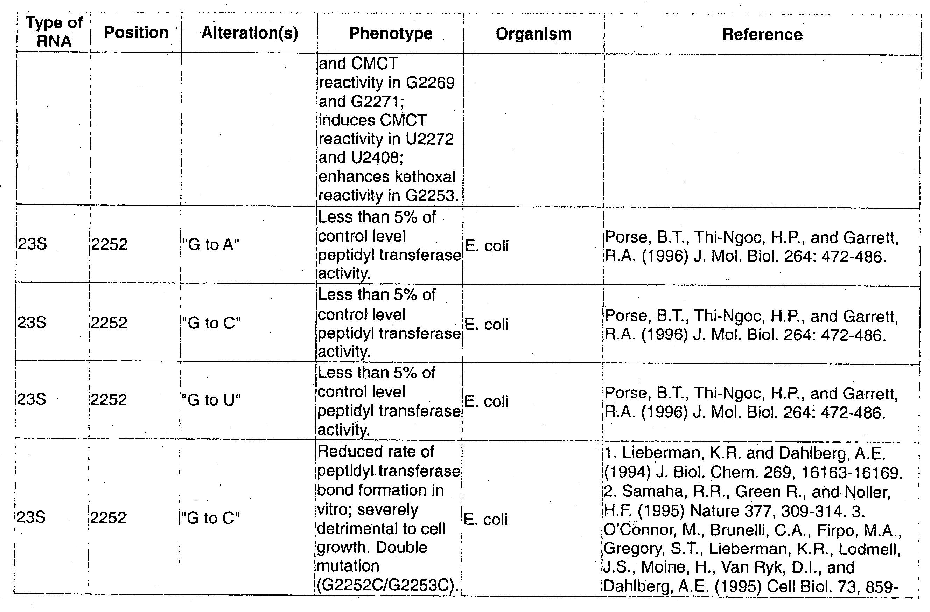

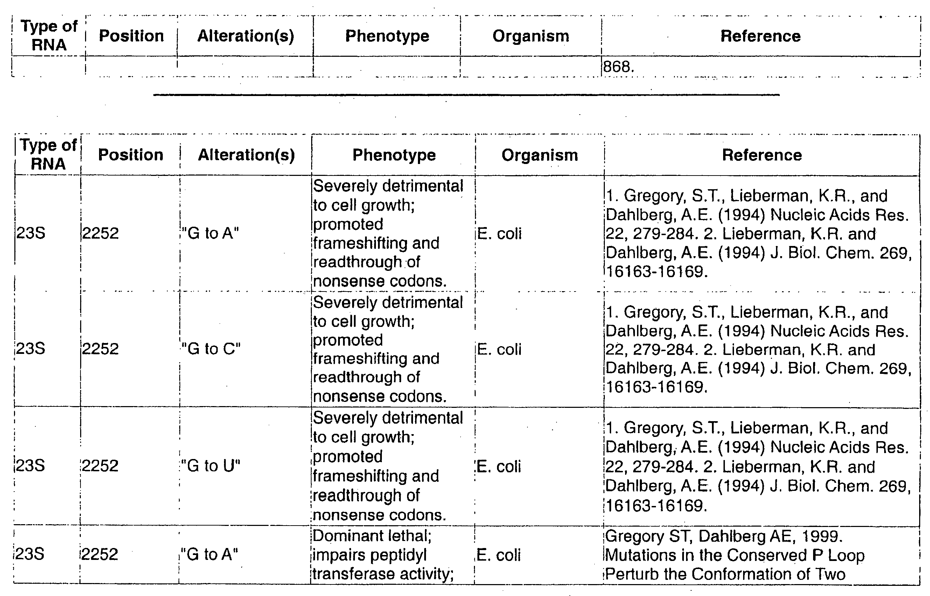

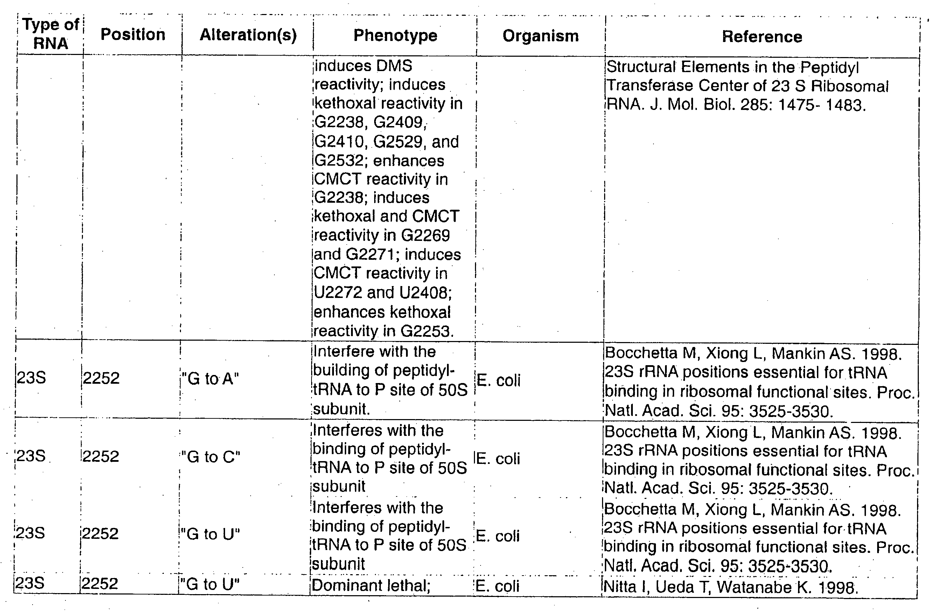

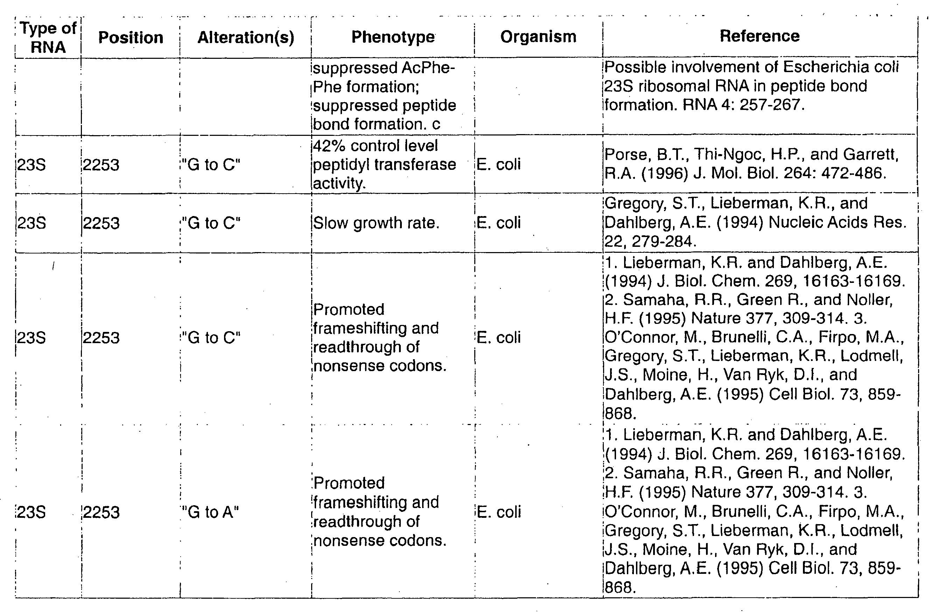

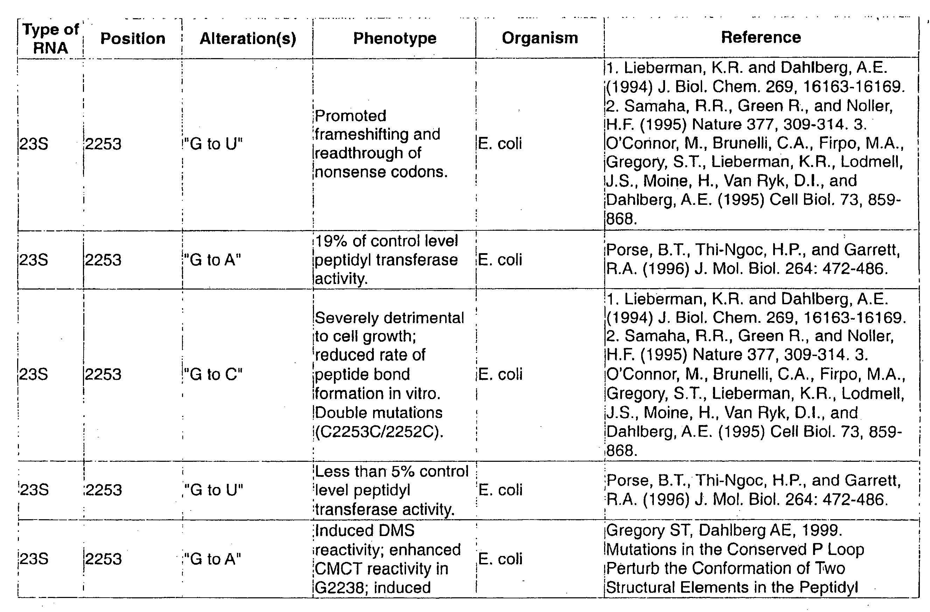

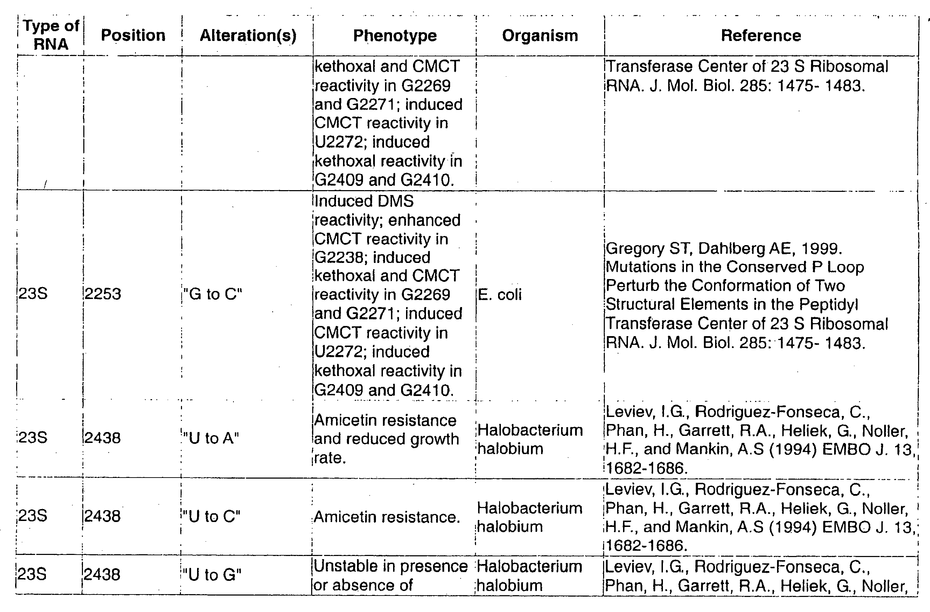

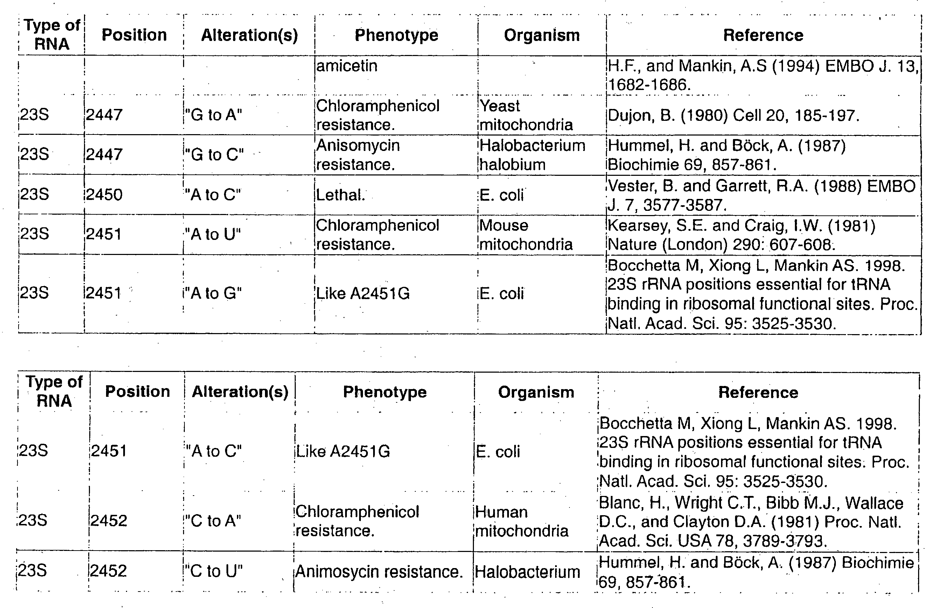

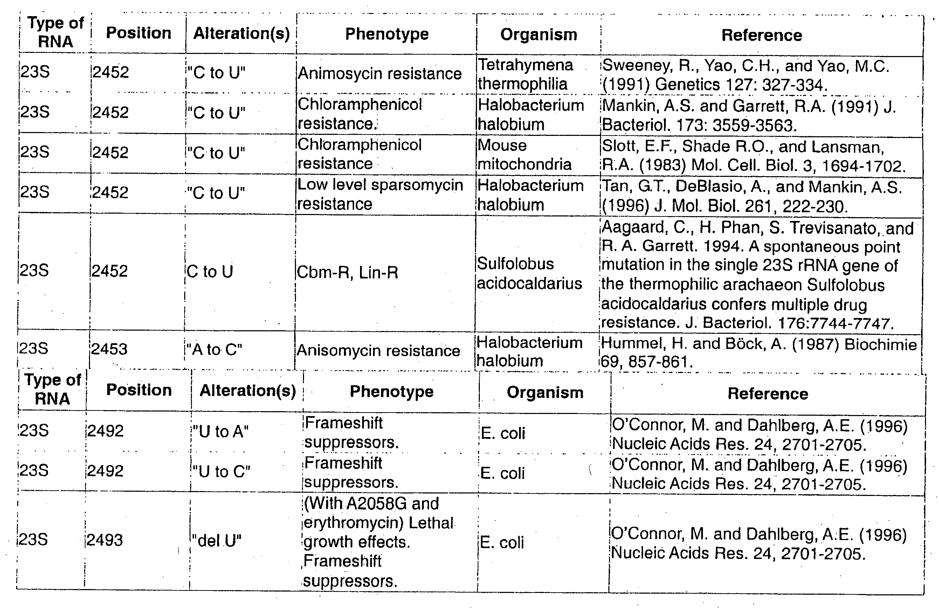

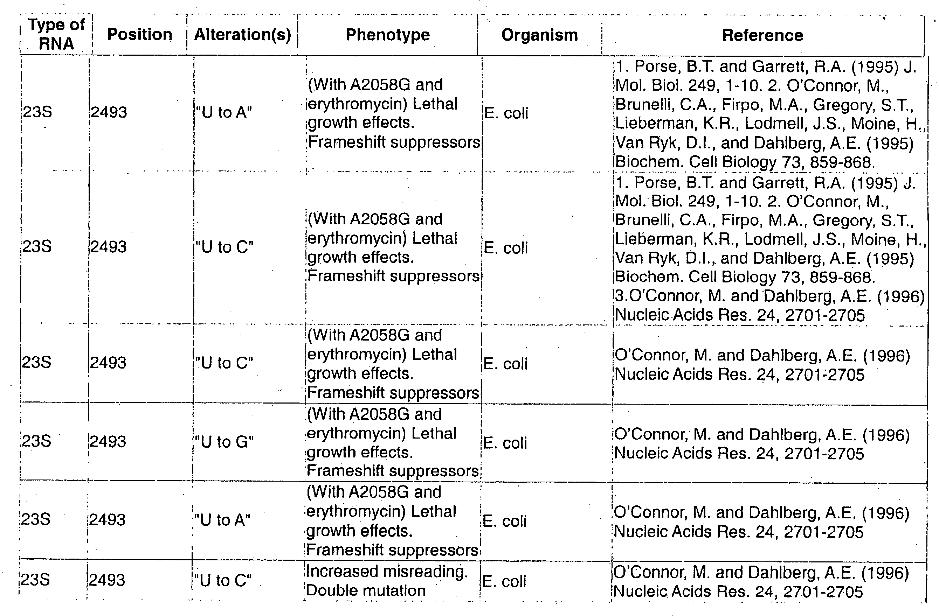

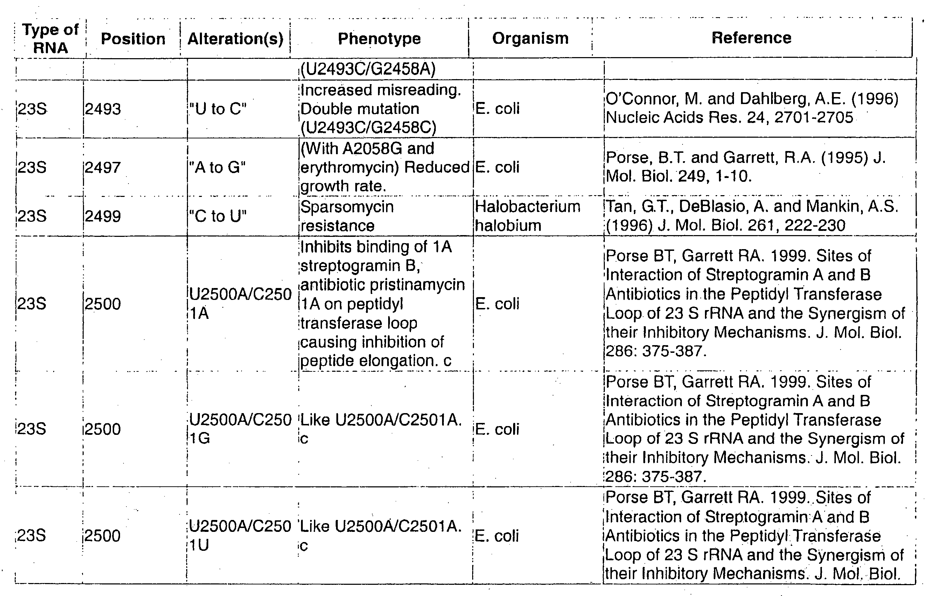

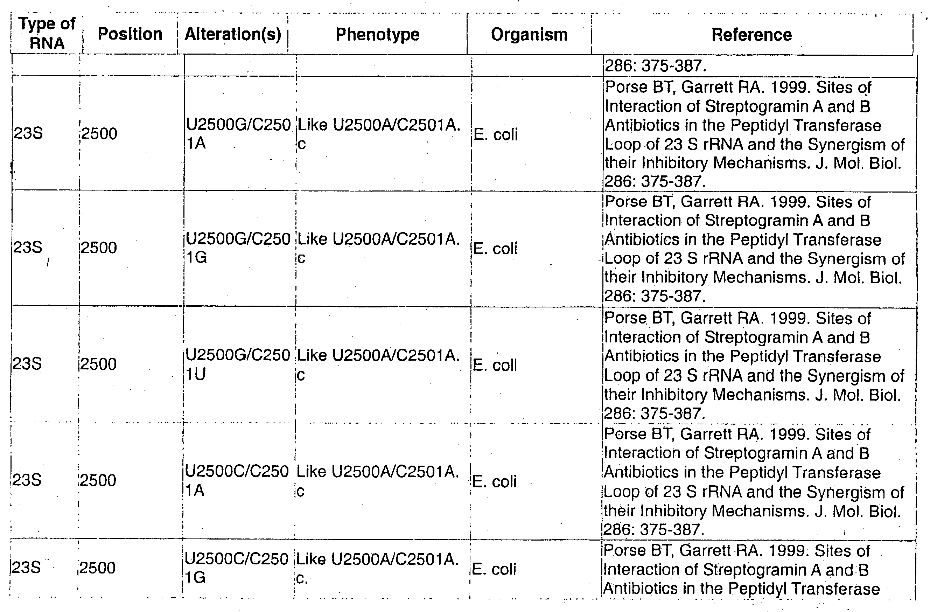

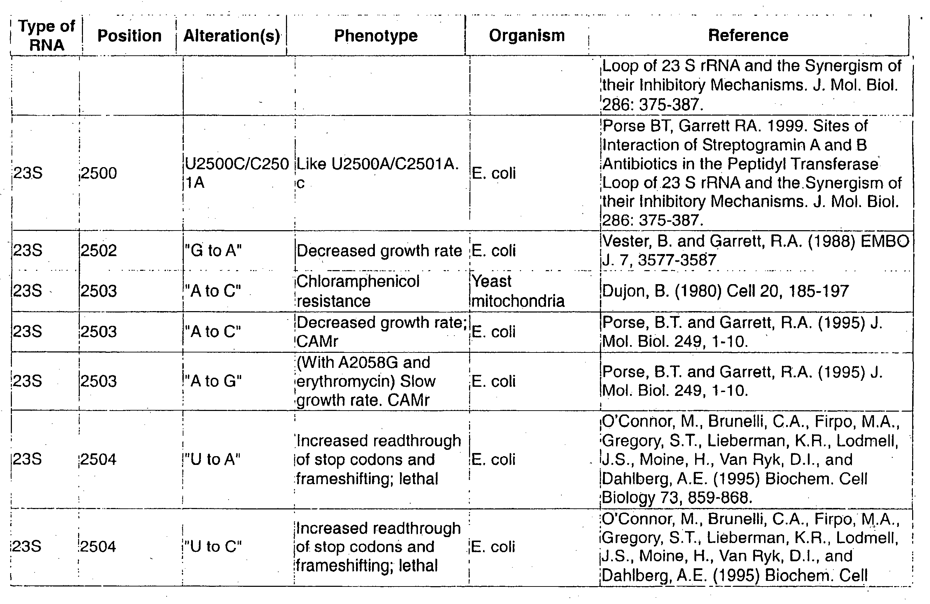

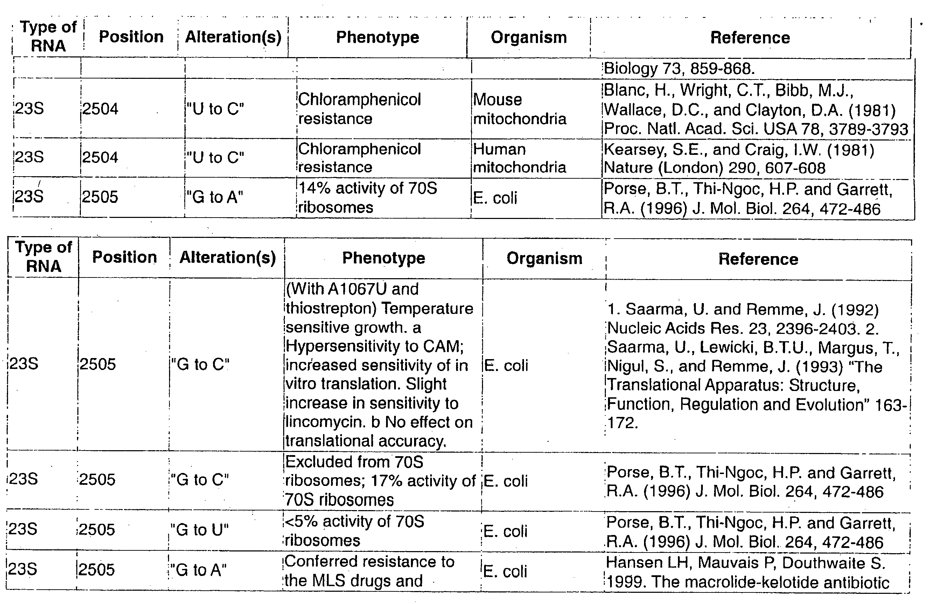

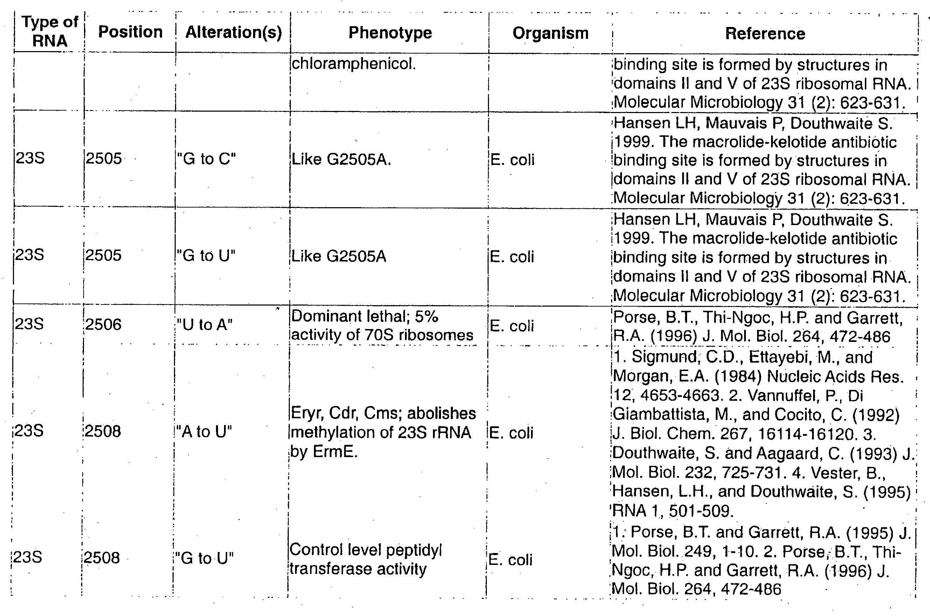

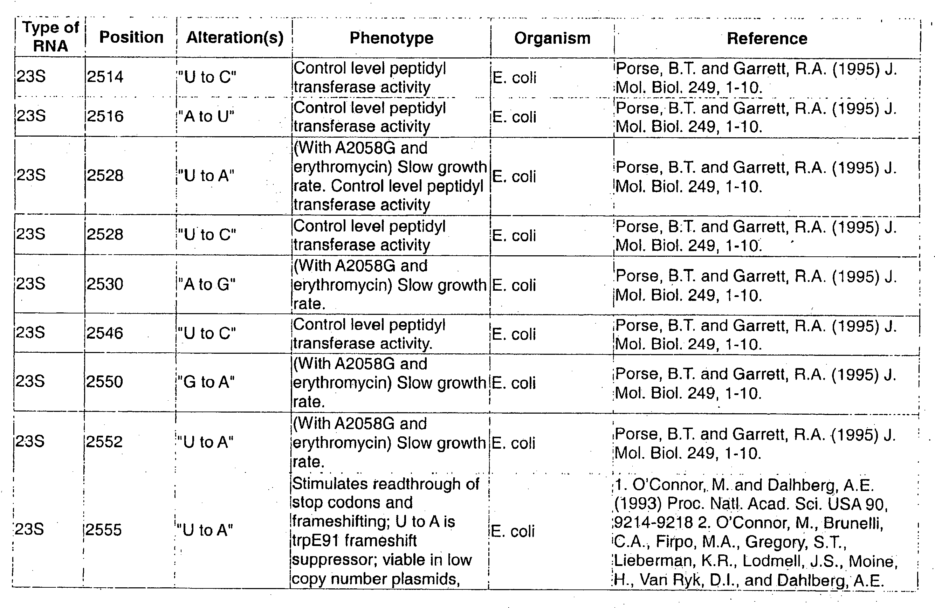

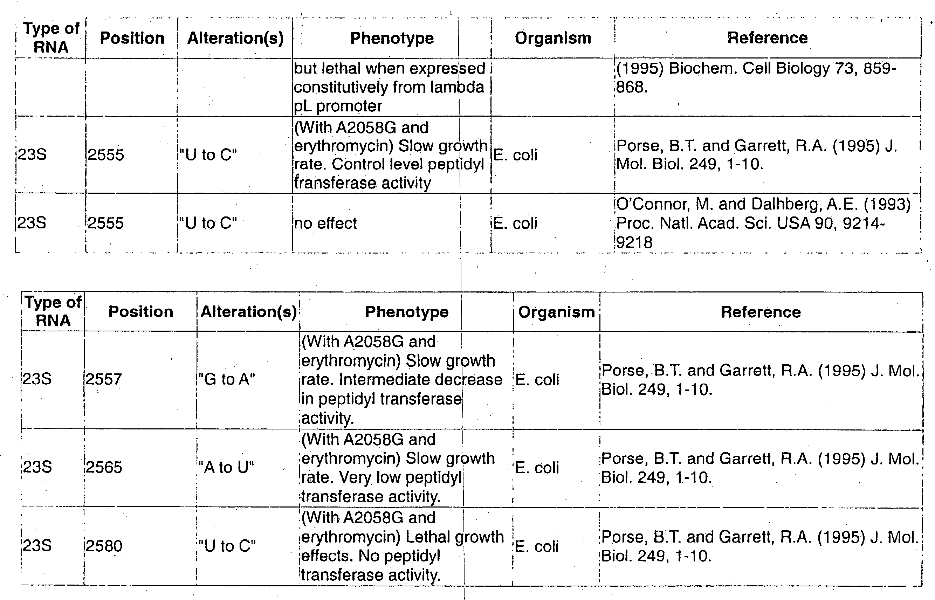

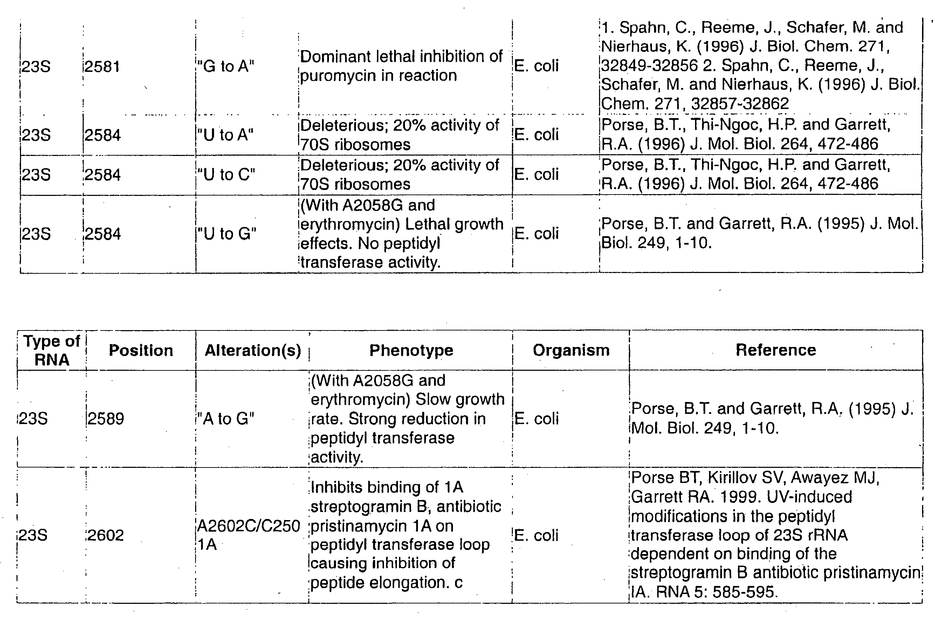

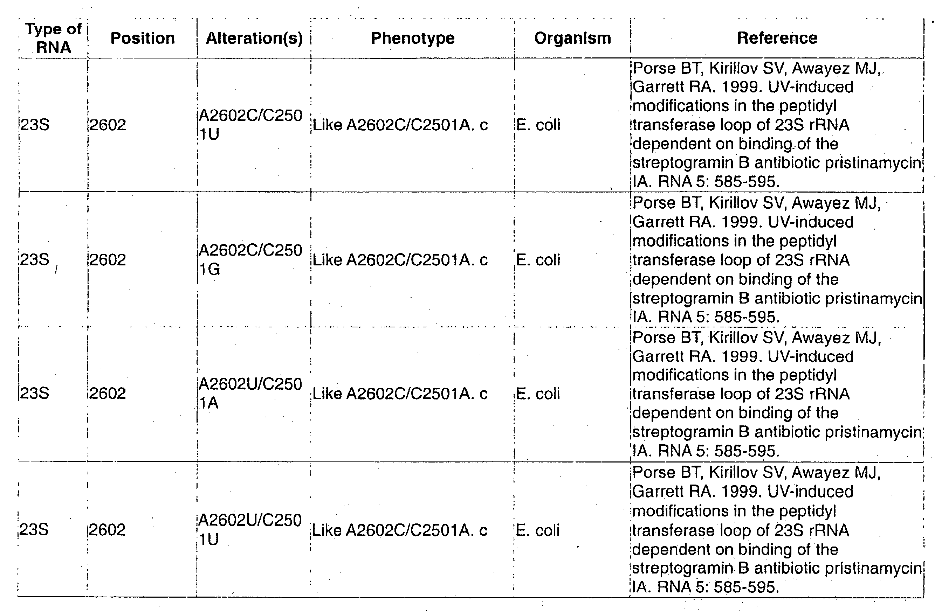

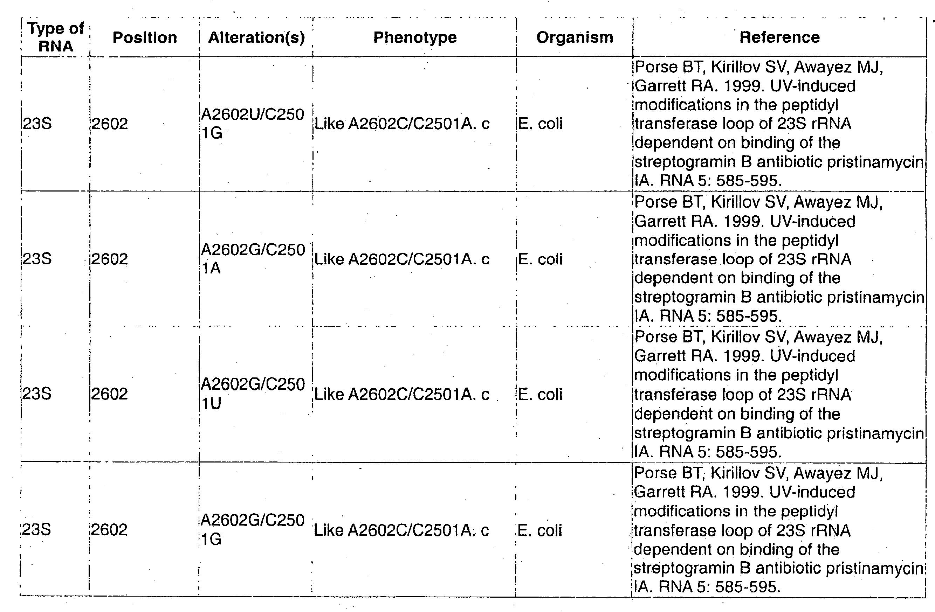

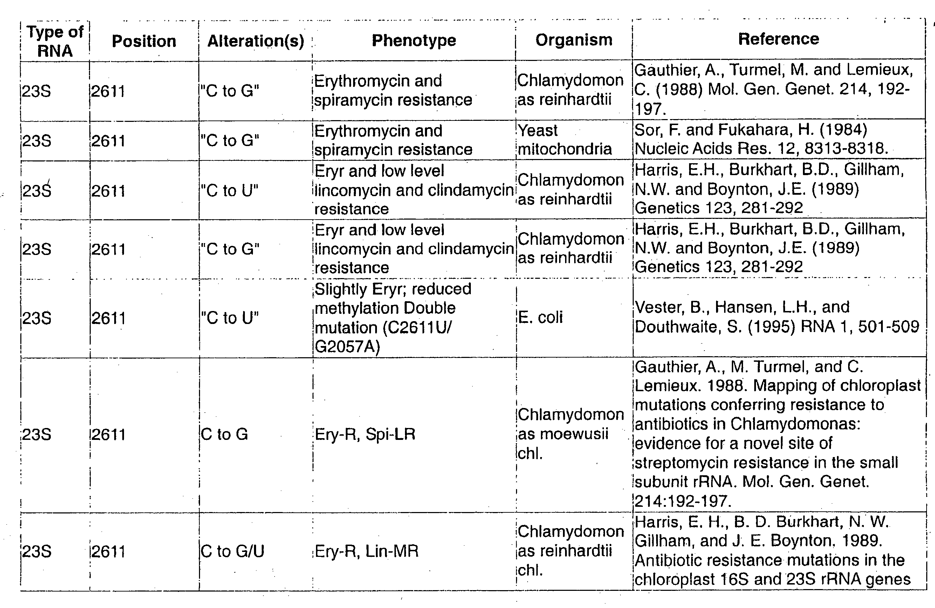

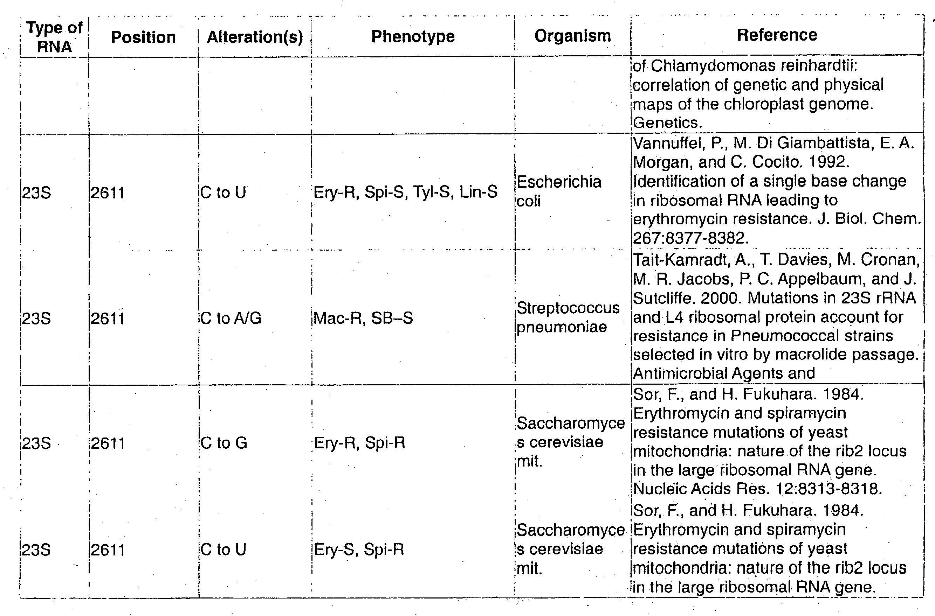

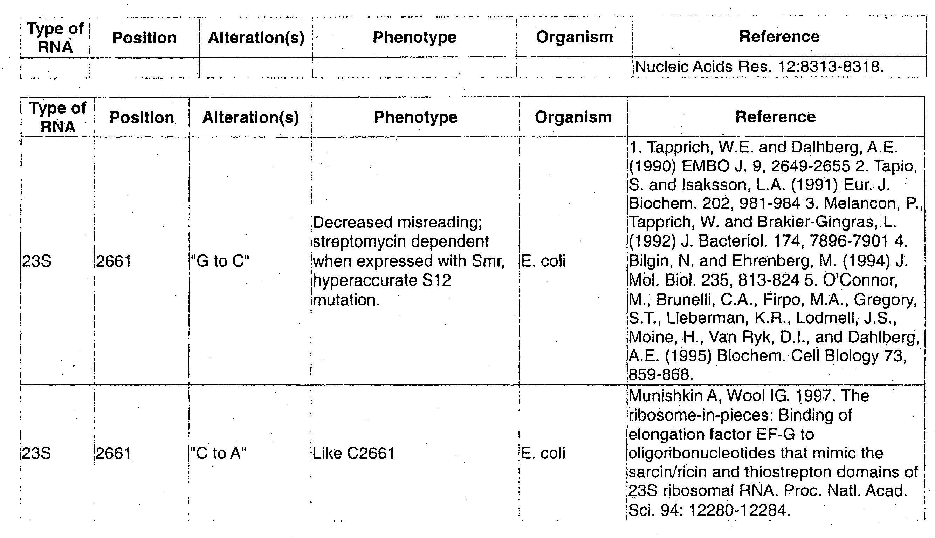

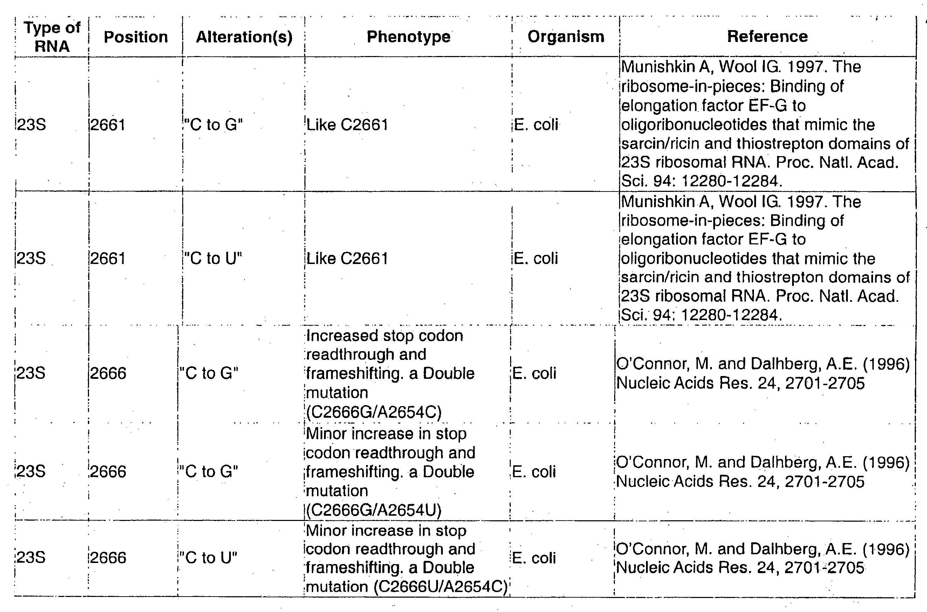

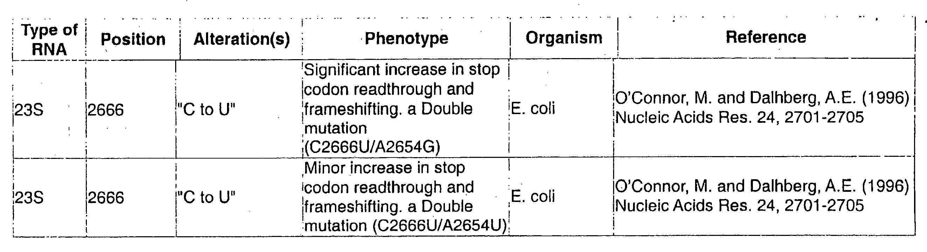

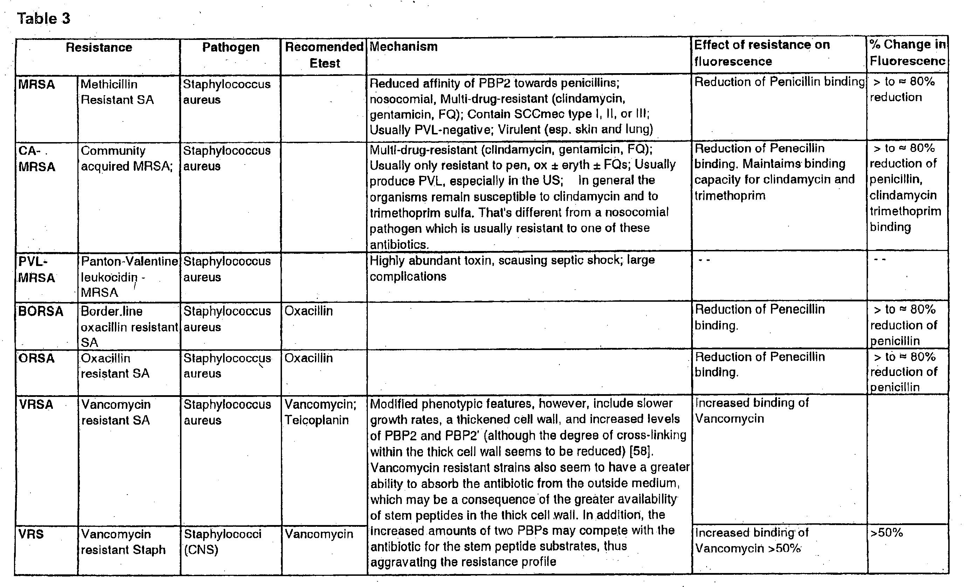

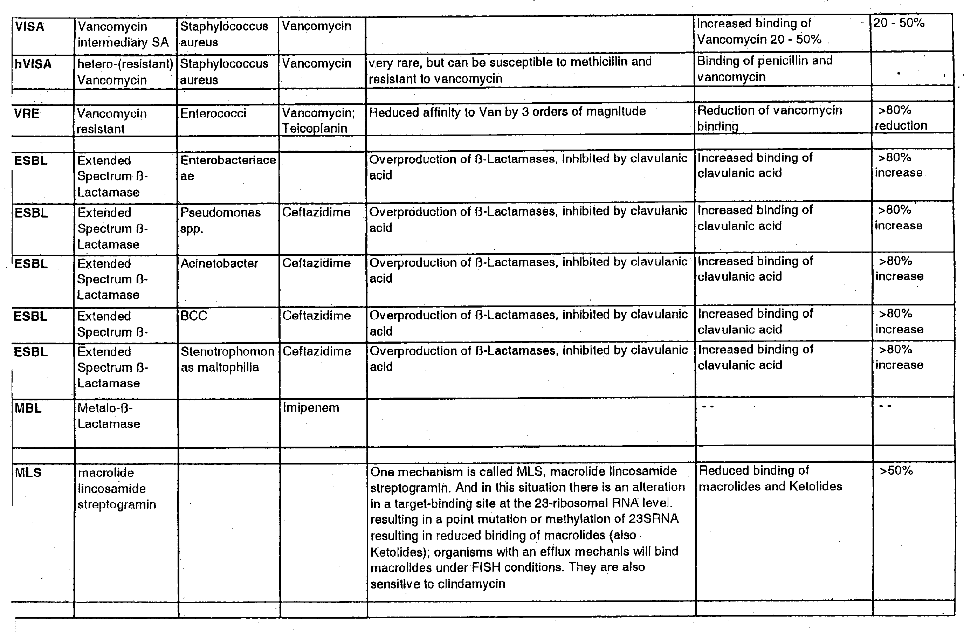

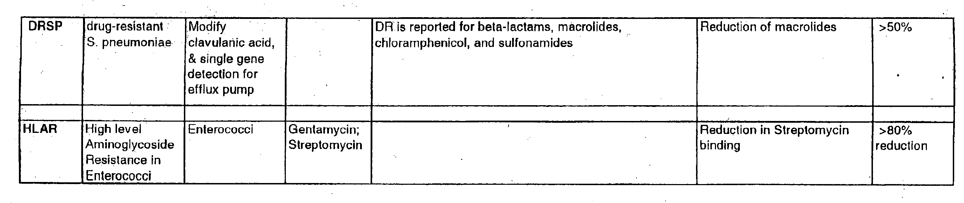

## Claims

1. Method for the simultaneous identification of an individual bacterial cell and detection of an antibiotic resistance in said individual bacterial cell in a biological sample, comprising the steps:
(a) contacting said individual bacterial cell with a first nucleic acid capable of selectively hybridizing with a nucleic acid in said bacterial cell under *in situ* conditions, wherein the first nucleic acid selectively hybridizes with the nucleic acid, and wherein the first nucleic acid is labelled with a first label,
(b) identifying the bacterial cell by the detection of the presence of the first label in said individual bacterial cell,
(c) contacting said same individual bacterial cell with at least one probe for detection of an antibiotic resistance in the bacterial cell under said same *in situ* conditions, wherein said at least one probe is selected from
(i) antibodies and fragments thereof, the antibodies and said fragments thereof being capable of selectively binding to a resistance factor, and wherein the antibody and said fragments thereof are labelled with a second label, wherein said individual bacterial cell is contacted with the antibody or/and said fragment thereof under conditions wherein the antibody or/and said fragment thereof selectively binds to the resistance factor,
(ii) second nucleic acids capable of hybridizing with an RNA coding for a resistance factor, wherein the second nucleic acid is labelled with a third label,
wherein said individual bacterial cell is contacted with the second nucleic acid under conditions wherein the second nucleic acid selectively hybridizes with the RNA,
(iii) knottins, cystine-knot proteins or/and aptamers capable of selectively binding to a resistance factor, and wherein the knottins, cystine-knot proteins or/and aptamers are labelled with a fourth label, wherein said individual bacterial cell is contacted with the knottin, cystine-knot protein or/and aptamer under conditions wherein the knottin, cystineknot protein or/and aptamer selectively binds to the resistance factor, and
(iv) nitrocefin, wherein said individual bacterial cell is contacted with said nitrocefin under conditions wherein beta-lactamase modifies said nitrocefin, and
(d) determining the antibiotic resistance of the bacterial cell by the detection of the presence of one or more of the following labels, the second label, the third label, the fourth label, or/and the modified nitrocefin in the individual bacterial cell,
wherein steps (b) and (d) are performed simultaneously in said individual bacterial cell.

2. The method of claim 1, wherein steps (a) and (c) are performed simultaneously or separately.

3. The method of claim 1 or 2, wherein steps (a), (b), (c) and (d) are performed simultaneously.

4. The method of any one of the preceding claims, comprising the induction of the antibiotic resistance in the bacterial cell.

5. The method of any one of the preceding claims, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters.

6. The method of any one of the claims 1 to 5. wherein the antibody includes a primary antibody capable of selectively binding to the resistance factor, and a secondary antibody labelled with the second label, wherein the secondary antibody is capable of selectively binding to the primary antibody.

7. The method of any one of the claims 1 to 5, wherein the antibody or/and fragment thereof and the second label are coupled to a bead.

8. The method of any one of the claims 1 to 5, wherein the knottih, cystineknot protein or/and aptamer and the fourth label are coupled to a bead.

9. The method of claim 7 or 8, wherein the bead has a diameter of 10 nm to 400 µm.

10. The method of any one of the claims 7 to 9, wherein the bead has a diameter of 50 µm to 400 µm, or 100 µm to 300 µm, or 150 µm to 250 µm, or 200 µm.

11. The method of any one of the claims 7 to 10, wherein in step (c) an aggregate is formed, said aggregate comprising more than one bacterial cell and at least one bead.

12. The method of any one of the claims 7 to 9, wherein the bead has a diameter of up to 100 nm, or up to 80 nm, or up to 60 nm, or up to 50 nm.

13. The method of any one of the claims 7 to 9 and 12, wherein in step (c), a plurality of beads is coupled to the bacterial cell.

14. The method according to any one the preceding claims, wherein the antibiotic is selected from the group consisting of aminoglycosides, carbacephems, carbapenems, cephalosporins, glycopeptides, macrolides, monobactams, penicillines, beta-lactam antibiotics, quinolones, bacitracin, sulfonamides, tetracyclines, streptogramines, chloramphenicol, clindamycin, and lincosamide.

15. The method according to any one of the preceding claims, wherein the resistance against a beta-lactam antibiotic is detected by an antibody or/and fragment thereof specifically binding to beta-lactamase or/and PBP2a binding protein.

16. The method according to any one of the preceding claims, wherein the resistance against a beta-lactam antibiotic is detected by a nucleic acid specifically hybridizing with an RNA encoding for beta-lactamase or/and PBP2a binding protein.

17. The method according to any one of the preceding claims, wherein the resistance against a beta-lactam antibiotic is detected by a knottin, a cystine knot protein or/and an aptamer specifically binding to beta-lactamase or/and PBP2a binding protein.

18. The method according to any one of the preceding claims, wherein the bacterial cell is a Methicillin Resistant *Staphylococcus aureus* (MRSA) or/and an Oxacillin Resistant *Staphylococcus aureus* (ORSA), and wherein the antibiotic resistance is detected by the expression of an altered Penicillin Binding Protein 2.

19. The method according to any one of the preceding claims, wherein the bacterial cell is a Methicillin Resistant *Staphylococcus aureus* (MRSA) or/and an Oxacillin Resistant *Staphylococcus aureus* (ORSA), and wherein the antibiotic resistance is detected by the expression of a mecA protein.

20. The method according to any one of the preceding claims, wherein the bacterial cell is selected from Vancomycin Resistant *Staphylococcus aureus* (VRSA), Vancomycin Resistant Staphylococcus (VRS), Vancomycin Resistant Enterococci (VRE) and Vancomycin Resistant Clostridium difficile (VRCD), and wherein the antibiotic resistance is detected by the expression of a peptide selected from vanA protein, vanB protein, vanC protein or/and modified peptidoglycans comprising a D-alanine-D-lactate C-terminus.

21. Use of a kit simultaneously identifying an individual bacterial cell and detecting an antibiotic resistance in said individual bacterial cell in a biological sample, wherein said kit is used in the method of any one of claims 14 to 20, said use comprising
(a) the use of a first nucleic acid capable of selectively hybridizing with a nucleic acid in said individual bacterial cell under *in situ* conditions, wherein the first nucleic acid is labelled with a first label, and
(b) the use of at least one probe for detection of an antibiotic resistance in said individual bacterial cell under said same *in situ* conditions, wherein said at least one probe is selected from
(i) antibodies and fragments thereof, wherein the antibodies and said fragments thereof are capable of selectively binding to a resistance factor, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters, and wherein the antibody and said fragments thereof are labelled with a second label,
(ii) second nucleic acids capable of hybridizing with an RNA coding for a resistance factor, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters, and wherein the second nucleic acid is labelled with a third label,
(iii) knottins, cystine-knot proteins or/and aptamers capable of selectively binding to a resistance factor, wherein the resistance factor is selected from PBP2a, β-lactamase, efflux transporters, wherein the efflux transporters are preferably selected from the group consisting of ATP-Binding Cassette (ABC) transporters, Major Facilitator Superfamily (MFS) transporters, Multidrug and Toxic Compound Extrusion (MATE) transporters and Resistance Nodulation Division (RND) transporters, and wherein the knottins, cystine-knot proteins or/and aptamers are labelled with a fourth label, and
(iv) nitrocefin which can be modified by beta-lactamase.

## Patentansprüche

1. Verfahren zum gleichzeitigen Identifizieren einer einzelnen Bakterienzelle und Nachweis einer Antibiotikaresistenz in dieser einzelnen Bakterienzelle in einer biologischen Probe, umfassend die Schritte:
(a) In Kontakt bringen dieser einzelnen Bakterienzelle mit einer ersten Nukleinsäure, die in der Lage ist, mit einer Nukleinsäure in dieser Bakterienzelle unter *in-situ -* Bedingungen selektiv zu hybridisieren, wobei die erste Nukleinsäure mit der Nukleinsäure selektiv hybridisiert, und wobei die erste Nukleinsäure mit einem ersten Marker markiert ist,
(b) Identifizieren der Bakterienzelle durch Nachweisen der Präsenz des ersten Markers in dieser einzelnen Bakterienzelle,
(c) in Kontakt bringen derselben einzelnen Bakterienzelle mit mindestens einer Sonde zum Nachweis einer Antibiotikaresistenz in der Bakterienzelle unter denselben *in-situ -* Bedingungen, wobei diese mindestens eine Sonde ausgewählt ist aus
(i) Antikörpern und deren Fragmenten, wobei die Antikörper und deren Fragmente in der Lage sind, selektiv an einen Resistenzfaktor zu binden, und wobei der Antikörper und dessen Fragmente mit einem zweiten Marker markiert sind,
wobei diese einzelne Bakterienzelle mit dem Antikörper und/oder dessen Fragment unter Bedingungen in Kontakt gebracht wird, unter denen der Antikörper und/oder dessen Fragment selektiv an den Resistenzfaktor bindet,
(ii) zweiten Nukleinsäuren, die in der Lage sind, mit einer RNA zu hybridisieren, welche einen Resistenzfaktor kodiert, wobei die zweite Nukleinsäure mit einem dritten Marker markiert ist,
wobei diese einzelne Bakterienzelle mit der zweiten Nukleinsäure unter Bedingungen in Kontakt gebracht wird, unter denen die zweite Nukleinsäure selektiv mit der RNA hybridisiert,
(iii) Knottins, Cystinknoten-Proteinen und/oder Aptameren, die in der Lage sind, selektiv an einen Resistenzfaktor zu binden, wobei die Knottins, Cystinknoten-Proteine und/oder Aptamere mit einem vierten Marker markiert sind, wobei diese einzelne Bakterienzelle mit dem Knottin, Cystinknoten-Protein und/oder Aptamer unter Bedingungen in Kontakt gebracht wird, unter denen das Knottin, Cystinknoten-Protein und/oder Aptamer selektiv an den Resistenzfaktor bindet, und
(iv) Nitrocefin, wobei diese einzelne Bakterienzelle mit dem Nitrocefin unter Bedingungen in Kontakt gebracht wird, unter denen Beta-Lactamase das Nitrocefin modifiziert, und
(d) Bestimmen der Antibiotikaresistenz der Bakterienzelle durch den Nachweis der Präsenz einer oder mehrerer der folgenden Marker: des zweiten Markers, des dritten Markers, des vierten Markers und/oder des modifizierten Nitrocefins in der einzelnen Bakterienzelle,
wobei die Schritte (b) und (d) gleichzeitig in dieser einzelnen Bakterienzelle durchgeführt werden.

2. Verfahren nach Anspruch 1, wobei die Schritte (a) und (c) gleichzeitig oder separat durchgeführt werden.

3. Verfahren nach Anspruch 1 oder 2, wobei die Schritte (a), (b), (c) und (d) gleichzeitig durchgeführt werden.

4. Verfahren nach einem der vorhergehenden Ansprüche, welches das Induzieren der Antibiotikaresistenz in der Bakterienzelle umfasst.

5. Verfahren nach einem der vorhergehenden Ansprüche, wobei der Resistenzfaktor ausgewählt ist aus PBP2a, β-Lactamase und Efflux-Transportern, wobei die Efflux-Transporter vorzugsweise aus der Gruppe, die aus ABC (ATP-Binding Cassette)-Transportern, MFS (Major Facilitator Superfamily)-Transportern, MATE (Multidrug and Toxic Compound Extrusion)-Transportern und RND (Resistance Nodulation Division)-Transportern besteht, ausgewählt ist.

6. Verfahren nach einem der Ansprüche 1 bis 5, wobei der Antikörper einen primären Antikörper, der in der Lage ist, selektiv an den Resistenzfaktor zu binden, und einen sekundären Antikörper, der mit dem zweiten Marker markiert ist, umfasst, wobei der sekundäre Antikörper in der Lage ist, selektiv an den primären Antikörper zu binden.

7. Verfahren nach einem der Ansprüche 1 bis 5, wobei der Antikörper und/oder dessen Fragment und der zweite Marker an ein Kügelchen gekoppelt sind.

8. Verfahren nach einem der Ansprüche 1 bis 5, wobei das Knottin, Cystinknoten-Protein und/oder Aptamer und der vierte Marker an ein Kügelchen gekoppelt sind.

9. Verfahren nach Anspruch 7 oder 8, wobei das Kügelchen einen Durchmesser von 10 nm bis 400 µm hat.

10. Verfahren nach einem der Ansprüche 7 bis 9, wobei das Kügelchen einen Durchmesser von 50 µm bis 400 µm, oder 100 µm bis 300 µm, oder 150 µm bis 250 µm, oder 200 µm hat.

11. Verfahren nach einem der Ansprüche 7 bis 10, wobei in Schritt (c) ein Aggregat gebildet wird, welches mehr als eine Bakterienzelle sowie mindestens ein Kügelchen umfasst.

12. Verfahren nach einem der Ansprüche 7 bis 9, wobei das Kügelchen einen Durchmesser von bis zu 100 nm, oder bis zu 80 nm, oder bis zu 60 nm, oder bis zu 50 nm hat.

13. Verfahren nach einem der Ansprüche 7 bis 9 und 12, wobei in Schritt (c) eine Vielzahl von Kügelchen an die Bakterienzelle gekoppelt ist.

14. Verfahren nach einem der vorhergehenden Ansprüche, wobei das Antibiotikum ausgewählt ist aus der Gruppe bestehend aus Aminoglycosiden, Carbacephemen, Carbapenemen, Cephalosporinen, Glycopeptiden, Macroliden, Monobactamen, Penicillinen, Beta-Lactam-Antibiotika, Quinolonen, Bacitracin, Sulfonamiden, Tetracyclinen, Streptograminen, Chloramphenicol, Clindamycin, und Lincosamid.

15. Verfahren nach einem der vorhergehenden Ansprüche, wobei die Resistenz gegen ein Beta-Lactam-Antibiotikum durch einen Antikörper oder dessen Fragment nachgewiesen wird, der oder das spezifisch an Beta-Lactamase und/oder PBP2a-bindendes Protein bindet.

16. Verfahren nach einem der vorhergehenden Ansprüche, wobei die Resistenz gegen ein Beta-Lactam-Antibiotikum durch eine Nukleinsäure nachgewiesen wird, die spezifisch mit einer RNA hybridisiert, die für Beta-Lactamase und/oder PBP2a-bindendes Protein kodiert.

17. Verfahren nach einem der vorhergehenden Ansprüche, wobei die Resistenz gegen ein Beta-Lactam-Antibiotikum durch ein Knottin, Cystinknoten-Protein und/oder Aptamer nachgewiesen wird, das spezifisch an Beta-Lactamase und/oder PBP2a-bindendes Protein bindet.

18. Verfahren nach einem der vorhergehenden Ansprüche, wobei die Bakterienzelle ein Methicillin-resistenter *Staphylococcus aureus* (MRSA) und/oder ein Oxacillin-resistenter *Staphylococcus aureus* (ORSA) ist, und wobei die Antibiotikaresistenz durch die Expression eines veränderten Penicillin-Bindungs-Protein 2 nachgewiesen wird.

19. Verfahren nach einem der vorhergehenden Ansprüche, wobei die Bakterienzelle ein Methicillin-resistenter *Staphylococcus aureus* (MRSA) und/oder ein Oxacillin-resistenter *Staphylococcus aureus* (ORSA) ist, und wobei die Antibiotikaresistenz durch die Expression eines mecA-Proteins nachgewiesen wird.

20. Verfahren nach einem der vorhergehenden Ansprüche, wobei die Bakterienzelle aus Vancomycin-resistentem *Staphylococcus aureus* (VRSA), Vancomycin-resistentem Staphylococcus (VRS), Vancomycin-resistenten Enterococci (VRE) und Vancomycin-resistentem Clostridium difficile (VRCD) ausgewählt ist, und wobei die Antibiotikaresistenz durch die Expression eines Peptids nachgewiesen wird, das aus vanA-Protein, vanB-Protein, vanC-Protein und/oder modifizierten Peptidoglycanen, die einen D-Alanin-D-Lactat-C-Terminus umfassen, ausgewählt ist.

21. Verwendung eines Kits zum gleichzeitigen Identifizieren einer einzelnen Bakterienzelle und Nachweis einer Antibiotikaresistenz in dieser einzelnen Bakterienzelle in einer biologischen Probe, wobei dieses Kit in dem Verfahren nach einem der Ansprüche 14 bis 20 eingesetzt wird, wobei diese Verwendung umfasst:
(a) die Verwendung einer ersten Nukleinsäure, die in der Lage ist, mit einer Nukleinsäure in dieser Bakterienzelle unter *in-situ -* Bedingungen selektiv zu hybridisieren, wobei die erste Nukleinsäure mit einem ersten Marker markiert ist, und
(b) die Verwendung mindestens einer Sonde zum Nachweis einer Antibiotikaresistenz in dieser einzelnen Bakterienzelle unter denselben *in-situ -* Bedingungen, wobei diese mindestens eine Sonde ausgewählt ist aus
(i) Antikörpern und deren Fragmenten, wobei die Antikörper und deren Fragmente in der Lage sind, selektiv an einen Resistenzfaktor zu binden, wobei der Resistenzfaktor aus PBP2a, β-Lactamase und Efflux-Transportern ausgewählt ist, wobei die Efflux-Transporter vorzugsweise aus der Gruppe, die aus ABC (ATP-Binding Cassette)-Transportern, MFS (Major Facilitator Superfamily)-Transportern, MATE (Multidrug and Toxic Compound Extrusion)-Transportern und RND (Resistance Nodulation Division)-Transportern besteht, ausgewählt ist, und wobei der Antikörper und die Antikörper-Fragmente mit einem zweiten Marker markiert sind,
(ii) zweiten Nukleinsäuren, die in der Lage sind, mit einer RNA zu hybridisieren, welche einen Resistenzfaktor kodiert, wobei der Resistenzfaktor aus PBP2a, β-Lactamase und Efflux-Transportern ausgewählt ist, wobei die Efflux-Transporter vorzugsweise aus der Gruppe, die aus ABC (ATP-Binding Cassette)-Transportern, MFS (Major Facilitator Superfamily)-Transportern, MATE (Multidrug and Toxic Compound Extrusion)-Transportern und RND (Resistance Nodulation Division)-Transportern besteht, ausgewählt ist, und wobei die zweite Nukleinsäure mit einem dritten Marker markiert ist,
(iii) Knottins, Cystinknoten-Proteinen und/oder Aptameren, die in der Lage sind, selektiv an einen Resistenzfaktor zu binden, wobei der Resistenzfaktor aus PBP2a, β-Lactamase und Efflux-Transportern ausgewählt ist, wobei die Efflux-Transporter vorzugsweise aus der Gruppe, die aus ABC (ATP-Binding Cassette)-Transportern, MFS (Major Facilitator Superfamily)-Transportern, MATE (Multidrug and Toxic Compound Extrusion)-Transportern und RND (Resistance Nodulation Division)-Transportern besteht, ausgewählt ist, und wobei die Knottins, Cystinknoten-Proteine und/oder Aptamere mit einem vierten Marker markiert sind, und
(iv) Nitrocefin, das durch Beta-Lactamase modifiziert werden kann.

## Revendications

1. Procédé pour l'identification simultanée d'une cellule bactérienne individuelle et pour la détection d'une résistance à un antibiotique dans ladite cellule bactérienne individuelle dans un échantillon biologique, comprenant les étapes consistant à :
(a) mettre en contact ladite cellule bactérienne individuelle avec un premier acide nucléique capable de s'hybrider de manière sélective avec un acide nucléique dans ladite cellule bactérienne dans des conditions in situ, le premier acide nucléique s'hybridant de manière sélective avec l'acide nucléique, et le premier acide nucléique étant marqué par un premier marqueur,
(b) identifier la cellule bactérienne par la détection de la présence du premier marqueur dans ladite cellule bactérienne individuelle,
(c) mettre en contact ladite même cellule bactérienne individuelle avec au moins une sonde pour la détection d'une résistance antibiotique dans la cellule bactérienne dans les mêmes conditions in situ, ladite au moins une sonde étant choisie parmi
(i) des anticorps et des fragments de ceux-ci, les anticorps et lesdits fragments de ceux-ci étant capables de se lier de manière sélective à un facteur de résistance, et l'anticorps et lesdits fragments de celui-ci étant marqués avec un second marqueur,
dans lequel ladite cellule bactérienne individuelle est mise en contact avec l'anticorps ou/et ledit fragment de celui-ci dans des conditions dans lesquelles l'anticorps ou/et ledit fragment de celui-ci se lie de manière sélective au facteur de résistance,
(ii) des seconds acides nucléiques capables de s'hybrider avec un ARN codant pour un facteur de résistance, le second acide nucléique étant marqué par un troisième marqueur,
dans lequel ladite cellule bactérienne individuelle est mise en contact avec le second acide nucléique dans des conditions dans lesquelles le second acide nucléique s'hybride de manière sélective avec l'ARN,
(iii) des protéines knottines, des protéines à noeud cystine ou/et des aptamères capables de se lier de manière sélective à un facteur de résistance, et les protéines knottines, les protéines à noeud cystine ou/et les aptamères étant marquées avec un quatrième marqueur, dans lequel ladite cellule bactérienne individuelle est mise en contact avec la protéine knottine, la protéine à noeud cystine ou/et l'aptamère dans des conditions dans lesquelles la protéine knottine, la protéine à noeud cystine ou/et l'aptamère se lie de manière sélective au facteur de résistance, et
(iv) la nitrocéfine, dans lequel ladite cellule bactérienne individuelle est mise en contact avec ladite nitrocéfine dans des conditions dans lesquelles une béta-lactamase modifie ladite nitrocéfine, et
(d) déterminer la résistance à un antibiotique de la cellule bactérienne par la détection de la présence d'un ou de plusieurs des marqueurs suivants, le second marqueur, le troisième marqueur, le quatrième marqueur, ou/et la nitrocéfine modifiée dans la cellule bactérienne individuelle,
dans lequel les étapes (b) et (d) sont effectuées simultanément dans ladite cellule bactérienne individuelle.

2. Procédé selon la revendication 1, dans lequel les étapes (a) et (c) sont effectuées simultanément ou séparément.

3. Procédé selon la revendication 1 ou la revendication 2, dans lequel les étapes (a), (b), (c) et (d) sont effectuées simultanément.

4. Procédé selon l'une quelconque des revendications précédentes, comprenant l'induction de la résistance à un antibiotique dans la cellule bactérienne.

5. Procédé selon l'une quelconque des revendications précédentes, dans lequel le facteur de résistance est choisi parmi PBP2a, β-lactamase, des transporteurs d'efflux, dans les transporteurs d'efflux sont de préférence choisis depuis le groupe constitué des transporteurs à cassette de fixation à l'ATP (ABC), des transporteurs d'une superfamille de facilitateurs majeurs (MFS), des transporteurs d'extrusion de composés multiples et toxiques (MATE) et des transporteurs de la division de nodulation de résistance (RND).

6. Procédé selon l'une quelconque des revendications 1 à 5, dans lequel l'anticorps inclut un anticorps primaire capable de se lier de manière sélective au facteur de résistance, et un anticorps secondaire marqué par le second marqueur, dans lequel l'anticorps secondaire est de se lier de manière sélective à l'anticorps primaire.

7. Procédé selon l'une quelconque des revendications 1 à 5, dans lequel l'anticorps ou/et le fragment de celui-ci et le second marqueur sont couplés à une bille.

8. Procédé selon l'une quelconque des revendications 1 à 5, dans lequel la protéine knottine, la protéine à noeud cystine ou/et l'aptamère et le quatrième marqueur sont couplés à une bille.

9. Procédé selon la revendication 7 ou la revendication 8, dans lequel la bille possède un diamètre compris entre 10 nm et 400 µm.

10. Procédé selon l'une quelconque des revendications 7 à 9, dans lequel la bille possède un diamètre compris entre 50 µm et 400 µm, ou entre 100 µm et 300 µm, ou entre 150 µm et 250 µm, ou de 200 µm.

11. Procédé selon l'une quelconque des revendications 7 à 10, dans lequel dans l'étape (c) un granulat est formé, ledit granulat comprenant plus d'une cellule bactérienne et au moins une bille.

12. Procédé selon l'une quelconque des revendications 7 à 9, dans lequel la bille possède un diamètre pouvant aller jusqu'à 100 nm, ou jusqu'à 80 nm, jusqu'à 60 nm, jusqu'à 50 nm.

13. Procédé selon l'une quelconque des revendications 7 à 9 et 12, dans lequel dans l'étape (c) une pluralité de billes est couplée à la cellule bactérienne.

14. Procédé selon l'une quelconque des revendications précédentes, dans lequel l'antibiotique est choisi parmi le groupe constitué d'aminoglycosides, de carbacéphèmes, de carbapénèmes, de céphalosporines, de glycopeptides, de macrolides, de monobactames, de pénicillines, de béta-lactamines, de quinolones, de la bacitracine, de sulfonamides, de tétracyclines, de streptogramines, du chloramphénicol, de la clindamycine et du lincosamide.

15. Procédé selon l'une quelconque des revendications précédentes, dans lequel la résistance à une béta-lactamine est détectée à l'aide d'un anticorps ou/et un fragment de celui-ci se liant de manière spécifique à une béta-lactamase ou/et à une protéine de liaison PBP2a.

16. Procédé selon l'une quelconque des revendications précédentes, dans lequel la résistance à une béta-lactamine est détectée à l'aide d'un acide nucléique s'hybridant de manière spécifique à un ARN codant pour une béta-lactamase ou/et à une protéine de liaison PBP2a.

17. Procédé selon l'une quelconque des revendications précédentes, dans lequel la résistance à une béta-lactamine est détectée à l'aide d'une knottine, d'une protéine à noeud cystine ou/et d'un aptamère se liant de manière spécifique à une béta-lactamase ou/et à une protéine de liaison PBP2a.

18. Procédé selon l'une quelconque des revendications précédentes, dans lequel la cellule bactérienne est un staphylocoque doré résistant à la méthycilline (MRSA) ou/et un staphylocoque doré résistant à l'oxacilline (ORSA), et dans lequel la résistance à un antibiotique est détectée par l'expression d'une protéine 2 de liaison à la pénicilline altérée.

19. Procédé selon l'une quelconque des revendications précédentes, dans lequel la cellule bactérienne est un staphylocoque doré résistant à la méthycilline (MRSA) ou/et un staphylocoque doré résistant à l'oxacilline (ORSA), et dans lequel la résistance à un antibiotique est détectée par l'expression d'une protéine mecA.

20. Procédé selon l'une quelconque des revendications précédentes, dans lequel est choisie parmi un staphylocoque doré résistant à la vancomycine (VRSA), un staphylocoque résistant à la vancomycine (VRS), un entérocoque résistant à la vancomycine (VRE), un clostridium difficile résistant à la vancomycine (VRCD), et dans lequel la résistance à un antibiotique est détectée par l'expression d'un peptide choisi parmi une protéine vanA, une protéine vanB, une protéine vanC ou/et des peptidoglycanes modifiés comprenant une extrémité C de D-alanine-D-lactate.

21. Utilisation d'un kit adapté pour l'identification simultanée d'une cellule bactérienne individuelle et pour la détection d'une résistance à un antibiotique dans ladite cellule bactérienne individuelle dans un échantillon biologique, dans laquelle ledit kit est utilisé dans le procédé selon l'une quelconque des revendications 14 à 20, ladite utilisation comprenant les étapes consistant à :
(a) l'utilisation d'un premier acide nucléique capable de s'hybrider de manière sélective avec un acide nucléique dans ladite cellule bactérienne dans des conditions in situ, le premier acide nucléique étant marqué par un premier marqueur, et
(b) l'utilisation d'au moins une sonde pour la détection d'une résistance à un antibiotique dans ladite cellule bactérienne dans les mêmes conditions in situ, ladite au moins une sonde étant choisie parmi
(i) des anticorps et des fragments de ceux-ci, les anticorps et lesdits fragments de ceux-ci étant capables de se lier de manière sélective à un facteur de résistance, le facteur de résistance étant choisi parmi PBP2a, β-lactamase, des transporteurs d'efflux, dans les transporteurs d'efflux sont de préférence choisis depuis le groupe constitué des transporteurs à cassette de fixation à l'ATP (ABC), des transporteurs d'une superfamille de facilitateurs majeurs (MFS), des transporteurs d'extrusion de composés multiples et toxiques (MATE) et des transporteurs de la division de nodulation de résistance (RND), l'anticorps et lesdits fragments de celui-ci étant marqués avec un second marqueur,
(ii) des seconds acides nucléiques capables de s'hybrider avec un ARN codant pour un facteur de résistance, le facteur de résistance étant choisi parmi PBP2a, β-lactamase, des transporteurs d'efflux, dans les transporteurs d'efflux sont de préférence choisis depuis le groupe constitué des transporteurs à cassette de fixation à l'ATP (ABC), des transporteurs d'une superfamille de facilitateurs majeurs (MFS), des transporteurs d'extrusion de composés multiples et toxiques (MATE) et des transporteurs de la division de nodulation de résistance (RND), et le second acide nucléique étant marqué par un troisième marqueur,
(iii) des protéines knottines, des protéines à noeud cystine ou/et des aptamères capables de se lier de manière sélective à un facteur de résistance, le facteur de résistance étant choisi parmi PBP2a, β-lactamase, des transporteurs d'efflux, dans les transporteurs d'efflux sont de préférence choisis depuis le groupe constitué des transporteurs à cassette de fixation à l'ATP (ABC), des transporteurs d'une superfamille de facilitateurs majeurs (MFS), des transporteurs d'extrusion de composés multiples et toxiques (MATE) et des transporteurs de la division de nodulation de résistance (RND), et les protéines knottines, les protéines à noeud cystine ou/et les aptamères étant marquées par un quatrième marqueur, et
(iv) la nitrocéfine qui peut être modifiée par une béta-lactamase.
